# EUROPÄISCHE PATENTSCHRIFT

(11) **EP 1 272 458 B1**
(45) Veröffentlichungstag und Bekanntmachung des Hinweises auf die Patenterteilung: **05.12.2007**
(21) Anmeldenummer: 01916979.6
(22) Anmeldetag: 09.02.2001
(51) Int. Cl.: C07C 271/22, C07C 271/34, C07C 275/26, C07C 279/18, C07D 295/18, A61K 31/325, A61P 7/02

(54) **ARGININ-MIMETIKA ALS FAKTOR XA-INHIBITOREN**
ARGININE MIMETICS AS FACTOR XA INHIBITORS
MIMETIQUES DE L'ARGININE UTILISES COMME INHIBITEURS DU FACTEUR XA

(30) Priorität: 09.02.2000 DE 10005631
(43) Veröffentlichungstag der Anmeldung: 08.01.2003
(73) Patentinhaber: Wilex AG, 81675 München (DE)
(72) Erfinder: MORODER, Luis, 82152 Martinsried (DE); SPERL, Stefan, 81549 München (DE); STÜRZEBECHER, Jörg, 99094 Erfurt-Rhoda (DE)
(74) Vertreter: Dey, Michael
(86) Internationale Anmeldenummer: PCT/EP2001/001423
(87) Internationale Veröffentlichungsnummer: WO 2001/058859

(56) Entgegenhaltungen:
- EP-A- 0 097 630
- EP-A- 0 513 675
- EP-A- 1 059 302
- WO-A-94/23758
- DE-A- 2 540 134
- STEFAN SPERL ET AL.: "Urethanyl-3-Amidinophenylalanine Derivatives as Inhibitors of Factor Xa. X-Ray Crystal Structure of a Trypsin/Inhibitor Complex and Modeling Studies" BIOLOGICAL CHEMISTRY, Bd. 381, April 2000 (2000-04), Seiten 321-329, XP001002861
- STEFAN SPERL ET AL.: "(4-Aminomethyl)phenylguanidine derivatives as nonpeptidic highly selective inhibitors of human urokinase" PROCEEDINGS OF THE NATIONAL ACADEMY OF SCIENCES OF USA., Bd. 97, Nr. 10, 9. Mai 2000 (2000-05-09), Seiten 5113-5118, XP002169711 NATIONAL ACADEMY OF SCIENCE. WASHINGTON., US ISSN: 0027-8424
- TORSTEN STEINMETZER ET AL.: "Pept. Proc. Am. Pept. Sym. 15th; A Novel Approach for SPPS of Compounds wit an Amidino Group in the C-terminal Residue Using Trityl Resins" 1999 , KLUWER , DORDRECHT, NL XP001002826 Seite 261, Spalte 262
- MARKUS BÖHM ET AL.: "Three-Dimensional Quantitative Structure-Activity Relationship Analysis Using Comparative Molecular Field Analysis and Comparative Molecular Similarity Indices Analysis to Elucidate Selectivity Differences of Inhibitors Binding to Trypsin, Thrombin, and Factor Xa" JOURNAL OF MEDICINAL CHEMISTRY., Bd. 42, 1999, Seiten 458-477, XP002169712 AMERICAN CHEMICAL SOCIETY. WASHINGTON., US ISSN: 0022-2623
- B. VOIGT ET AL.: "Synthese von N-alpha-Benzyloxycarbonyl-4-amidinophenyla laninamiden als thrombininhibitoren" PHARMAZIE., Bd. 40, Nr. 5, 1985, Seiten 305-306, XP002169713 BERLIN DD
- J. STÜRZEBECHER ET AL.: "Synthetische Inhibitoren der Serinproteasen" PHARMAZIE., Bd. 42, Nr. 2, 1987, Seiten 114-116, XP002169714 VEB VERLAG VOLK UND GESUNDHEIT. BERLIN., DD ISSN: 0031-7144
- HIDEAKI TSUNEMATSU ET AL.: "A New beta-Naphthylamide Substrate of p-Guanidino-L-Phenylalanine for Trypsin and Related Enzymes" JOURNAL OF BIOCHEMISTRY., Bd. 98, Nr. 6, Dezember 1985 (1985-12), Seiten 1597-1602, XP002169715 JAPANESE BIOCHEMICAL SOCIETY, TOKYO., JP ISSN: 0021-924X
- R. ALAN CHRUSCIEL ET AL.: "4-Acetamidophenyl Esters and 4-Acetamidoanilides of L-Arginine, p-Guanidino-L-phenylalanine, L-Lysine, N2-[D-Fructos-3-O-yl and D-Glucos-3-O-yl]acetyl-L-lysine as Potential Acrosin Inhibitors" TETRAHEDRON, (INCL TETRAHEDRON REPORTS), Bd. 47, Nr. 42, 21. Oktober 1991 (1991-10-21), Seiten 8831-8854, XP002169716 OXFORD GB
- DANIEL A. PEARSON ET AL.: "Thrombus Imaging Using Techneticum-99m-Labeled High-potency GPIIb/IIIa Receptor Antagonists. Chemistry and Initial Biological Studies" JOURNAL OF MEDICINAL CHEMISTRY., Bd. 39, Nr. 7, 1996, Seiten 1372-1382, XP002169717 AMERICAN CHEMICAL SOCIETY. WASHINGTON., US ISSN: 0022-2623
- YUSUKE SASAKI ET AL.: "Studies on Analgesic Oligopeptides. II. Structure-Activity Relationship among Thirty Analogs of a Cyclic Dipeptide, Cyclo(-Tyr-Arg-)" CHEMICAL AND PHARMACEUTICAL BULLETIN., Bd. 30, Nr. 12, Dezember 1982 (1982-12), Seiten 4435-4443, XP002169718 PHARMACEUTICAL SOCIETY OF JAPAN. TOKYO., JP ISSN: 0009-2363
- BERNHARD GABRIEL ET AL.: "DEsign of Benzamidine-Type Inhibitors of Factor Xa" JOURNAL OF MEDICINAL CHEMISTRY., Bd. 41, Nr. 22, 1998, Seiten 4240-4250, XP002169719 AMERICAN CHEMICAL SOCIETY. WASHINGTON., US ISSN: 0022-2623

## Beschreibung

Die Erfindung betrifft allgemein eine neue Art von Arginin-Mimetika, die Inhibitoren des Faktors Xₐ sind; pharmazeutische Zusammensetzungen, die diese Mimetika enthalten; sowie die Verwendung dieser Arginin-Mimetika zur Herstellung von Mitteln zur antithrombotischen Therapie.

An der Blutgerinnungskaskade beteiligte Proteine wie Thrombin sind seit jeher potentielle Ziele bei der Behandlung von Gefäßerkrankungen, um durch deren Inhibierung thrombotische Gefäßverschlüsse zu vermeiden bzw. um thrombotisch verschlossene Blutgefäße wieder zu eröffnen. Die Verwendung von herkömmlichen Antikoagulationsmitteln, die Thrombin-Inhibitoren enthalten, ist problematisch, da durch sie die Wahrscheinlichkeit von Blutungskomplikationen erhöht wird (G.J. Phillipides und J. Loscalzo (1996), Coronary Artery Dis. 7, 497-507). Ferner wird durch eine direkte Thrombin-Inhibierung nicht die Thrombin-Produktion aus Prothrombin unterbrochen. Somit ist in diesem Fall eine kontinuierliche Zuführung von größeren Dosen an Inhibitor erforderlich, um *in vivo* einen antithrombotischen Effekt aufrecht zu erhalten.

Bei der Suche nach neuen antithrombotischen Mitteln wurde somit die Inhibierung des Blutgerinnungs-Faktors Xₐ zu einem Haupttarget für die Wirkstoffentwicklung. Der Faktor Xₐ ist eine Trypsin-artige Serinprotease, die das Zymogen Prothrombin in seine aktive Form, Thrombin, umwandelt. Damit kann durch die Inhibierung des Faktors Xₐ die Bildung von Thrombin vermieden werden, während ein für die primäre Hämostase erforderliches Niveau an Thrombin-Aktivität aufrecht erhalten wird (F. Al-Obeidy und J.A. Ostrem (1998), Drug Discovery Today, 3, 223-231).

Gabriel et al., Journal of Medicinal Chemistry, 41(1998), 4240-4250, beschreibt razemische Para-Benzolsulfonylglycylphenylalanin-Derivate als Faktor Xa-Inhibitoren. Die Verbindungen weisen eine peptidische Verknüpfung der zwei Aminosäuremoleküle Phenlyalanin und Glycin auf. Aufgrund von Röntgenstrukturdaten wird dem Glycinrest die Funktion zugewiesen, die Restgruppen optimal in den Bindungstaschen des Enzyms zu positionieren. Als Linker zwischen den funktionellen Gruppen am N- und C-Terminus fungiert bei den dort beschriebenen Inhibitoren eine Aminosäure.

Gegenwärtig sind eine Vielzahl von Faktor Xₐ-Inhibitoren bekannt. Die Entwicklung einer Reihe dieser faktor Xₐ-Inhibitoren basiert auf der Konservierung des Strukturmotivs Gly-Arg (wobei Gly der P2-Rest und Arg der P1-Rest ist, d.h., Gly bindet in die S2-Tasche und Arg in die S1-Tasche des Faktor Xₐ-Proteins) an der Spaltstelle des Prothrombins durch Faktor Xₐ. Dabei wurde eine Vielzahl von Synthesen für Faktor Xₐ-Inhibitoren beschrieben, bei denen als Mimetikum für den Arg-Rest ein PhenylalaninRest eingesetzt wird, der an seinem Phenylring mit einer basischen Amidinogruppe substituiert ist (J. Stürzebecher et al. (1989), Thromb. Res. 54, 245-252). Es wurde gezeigt, daß die bislang wirksamsten Faktor Xₐ-Inhibitoren in dieser Reihe Derivate von 3'-Amidinophenylalanin sind.

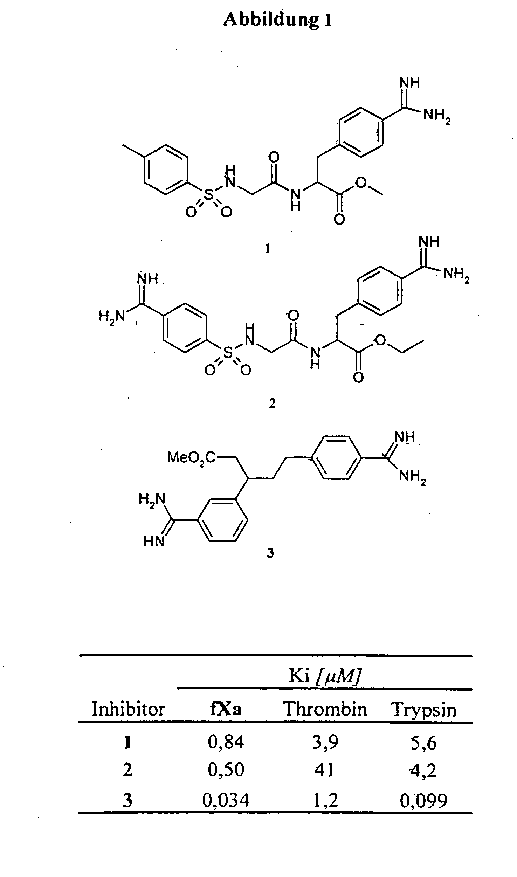

Ausgehend von der Leitstruktur N*^{a}*-Tosylglycyl-D,L-3-amidinophenylalaninalkylester (Verbindung 1 in Abb. 1; J. Stürzebecher et al., vide supra) wurde eine Vielzahl von peptidischen Bisbenzamidinverbindungen entwickelt: Der stärkste Faktor Xₐ-Inhibitor {Kᵢ = 0,5 *µ*M) aus dieser Reihe ist N°-4-Amidinobenzolsulfonylglycyl-D,L-4-amidino-phenylalaninethylester (Verbindung 2 in Abb. 1; B. Gabriel et al. (1998), J. Med. Chem. 41, 4240-4250), der "invers" an den Faktor Xₐ bindet: Seine 4'-Amidinobenzolsulfonylgruppe liegt in der S1-Tasche von Faktor Xₐ, während der Rest des Moleküls mit dem Glycyl-Spacer in die hydrophoben S3/S4-Bindungsstellen hineinragt, wodurch zusätzliche Wechselwirkungen mit dem elektronegativen Hohlraum, gebildet durch die Carbonylsauerstoffe von Lys 96, Thr 98, Glu 97 und dessen Carboxylatgruppe, hinter dem hydrophoben S3/S4-Bereich möglich werden.

Gemäß dem Stand der Technik stellt die Gruppe, die N-terminal mit einem Amidinophenylalaninrest oder einem anderen Arginin-Mimetikum verknüpft ist, den P3/P4-Rest des potentiellen Faktor Xₐ-Inhibitors dar, wobei die -N-terminal verknüpfte Gruppe vorzugsweise über einen Glycin-Spacer und eine Sulfonamidgruppe mit dem Arginin-Mimetikum verbunden ist.

Inzwischen wurden nicht-peptidische Bisbenzamidin-Verbindungen mit deutlich verbesserten Inhibitoreigenschaften (K₁=34 nM) erhalten, die durch einen kürzeren Abstand zwischen den zwei aromatischen Gruppen gekennzeichnet sind (T.P. Maduskuie et al. (1998), J. Med. Chem. 41, 53-62). Aufgrund von Modelling-Untersuchungen wird angenommen, daß ein derartiger Inhibitor 3 (siehe Abbildung 1) mit der m-Benzamidingruppierung in die S1-Tasche hineinreicht und dort mit dem Asp 189-Rest in Wechselwirkung tritt, und mit der p-Benzamidingruppe in die S4-Arylbindungstasche hineinreicht, wo sie in Kation-π- und hydrophobe Wechselwirkungen mit den umgebenen Resten Phe 174, Tyr 99 und Trp 215 tritt.

Vor diesem Hintergrund ist es eine Aufgabe der Erfindung, neue hocheffiziente und hochspezifische Inhibitoren des Faktors Xₐ bereitzustellen.

Des weiteren ist es eine wichtige Aufgabe der Erfindung, Möglichkeiten der Verwendung der erfindungsgemäßen Verbindungen zur Herstellung eines Mittels zur antithrombotischen Therapie aufzuzeigen.

Weitere Aufgaben und Vorteile der Erfindung ergeben sich aus der folgenden Beschreibung.

Diese Aufgaben werden durch die Gegenstände der unabhängigen Ansprüche, insbesondere basierend auf der Bereitstellung der erfindungsgemäßen Verbindungen gemäß der Strukturformel I, gelöst.

Vorteilhafte Ausgestaltungen sind in den Unteransprüchen beschrieben.

Insbesondere wird ein hocheffizienter und hochselektiver Faktor Xₐ-Inhibitor bereitgestellt, der ein Arginin-Mimetikum umfaßt, das einen N-terminalen Rest und einen C-terminalen Rest aufweist, wobei die Konformation des Inhibitors zwischenmolekulare

Wechselwirkungen zwischen dem C-terminalen Rest und der S3/S4-Tasche des Faktor Xₐ-Proteins ermöglicht. Ein derartiger Bindungsmodus, bei dem der C-terminale Rest in die S3/S4-Bindungstasche des Faktor Xₐ-Proteins reicht, ist besonders vorteilhaft, da bei dem erfindungsgemäßen Faktor Xₐ-Inhibitor neben dem N-terminalen Rest auch der C-terminale Rest des Arginin-Mimetikums in zwischenmolekulare Wechselwirkungen mit dem Faktor Xₐ-Protein tritt. Folglich kann der erfindungsgemä&e Faktor Xₐ-Inhibitor sowohl am N-terminalen Rest als auch am C-terminalen Rest optimiert werden, so daß Inhibitorstärken bereitgestellt werden können, die denjenigen des Stands der Technik deutlich überlegen sind.

Ein derartiger Bindungsmodus ist überraschend, da alle bislang aus dem Stand der Technik bekannten, auf Arginin-Mimetika basierenden Faktor Xₐ-Inhibitoren Substrat-artig binden. Bei der Substrat-artigen Bindung bindet das Arginin-Mimetikum in die S1-Tasche, während der Rest, der N-terminal mit dem Arginin-Mimetikum über einen potentiellem P2-Rest wie einen Gly-Spacer oder dergleichen verknüpft ist, in die S3- bzw. S4-Tasche reicht. Bei dem erfindungsgemäßen Faktor Xₐ-Inhibitor bindet das Arginin-Mimetikum ebenfalls in die S1-Tasche, aber im-Gegensatz zu den im Stand der Technik bekannten Faktor Xₐ-Inhibitoren reicht der C-terminal an des Arginin-Mimetikum gebundene Rest und nicht der N-terminal gebundene Rest in die S3- bzw- S4-Tasche des Faktor Xₐ-Proteins.

Im Rahmen der vorliegenden Erfindung besitzen die folgenden Begriffe die folgenden Bedeutungen, falls nicht ausdrücklich anders festgelegt:

Ein N-terminaler Rest des Arginin-Mimetikums bzw. ein N-terminal mit dem Arginin-Mimetikum verknüpfter Rest ist ein Rest, der mit dem Arginin-Mimetikum über das N*ₐ*-Atom der N-terminalen Aminogruppe des Arginin-Mimetikums bzw. über die, der Aminogruppe des nicht-modifizierten Arginins entsprechende Gruppe des Arginin-Mimetillums verbunden ist.

Entsprechend wird im Rahmen der vorliegenden Erfindung unter einem C-terminalen Rest des Arginin-Mimetikums bzw. einem C-terminal mit dem Arginin-Mimetikum verknüpften Rest ein Rest verstanden, der mit dem Arginin-Mimetikum über das C-terminale C-Atom der Carboxylgruppe des Arginin-Mimetikums bzw. über die der Carboxylgruppe des nicht-modifizierten Arginins entsprechende Gruppe des Arginin-Mimetikums verbunden ist.

Zwischenmolekulare Wechselwirkungen im Rahmen der vorliegenden Erfindung sind alle Formen von van-der-Waals-Wechselwirkungen wie elektrostatische Wechselwirkungen zwischen geladenen Resten des Inhibitors und entgegengesetzt geladenen Gruppen des Faktor Xₐ-Proteins, Wechselwirkungen zwischen polaren Gruppen des Inhibitors und entgegengesetzt polarisierten Gruppen des Faktor Xₐ-Proteins sowie hydrophobe Wechselwirkungen zwischen unpolaren Gruppen des Inhibitors und des Faktor Xₐ-Proteins und dergleichen, aber auch Wasserstoffbrücken-Bindungen zwischen dem Inhibitor und dem Faktor Xₐ-Protein.

Unter einem Arginin-Mimetikum wird im Zusammenhang der vorliegenden Erfindung eine Verbindung verstanden, die dieselben oder ähnliche funktionelle Charakteristika wie Arginin aufweist, z.B. eine Seitenkette mit einer positiven Ladung bei physiologischem pH, wie es für die Guanidinium-Gruppe der Seitenkette von Arginin charakteristisch ist. So kann ein Arginin-Mimetikum ein Aminosäure-Analogon von Arginin sein, d.h., eine Verbindung, bei der die N-terminale Aminogruppe, die C-terminale Carboxygruppe und/oder die Seitenkette des Arginins chemisch modifiziert wurde.

Bei der vorliegenden Erfindung können als Arginin-Mimetika insbesondere Aminosäure-Analoga eingesetzt werden, bei denen die Seitenkette einen substituierten oder nicht substituierten, gesättigten oder ungesättigten, carbocyclischen oder heterocyclischen Rest umfaßt. Ein derartiger Ring ist vorzugsweise ein Phenylring, kann aber auch ein Pyridin- oder ein Piperidinring oder eine anderere gesättigte oder ungesättigte oder aromatische, carbocyclische oder heterocyclische Gruppe sein, wobei das oder die Heteroatome Stickstoff, Sauerstoff und/oder Schwefel sein können.

Substituenten eines derartigen, vorstehend genannten carbocyclischen oder heterocyclischen Restes sind vorzugsweise basische Substituenten wie Amidino-, Guanidino-, Amino-, Alkylamino-, Aminoalkyl-, Amid-Substituenten und dergleichen. Ferner können auch polare Substituenten wie Halogene, z.B. Chlor, Hydroxyl oder Alkoxy vorteilhaft eingesetzt werden. Die vorstehend genannten carbocyclischen oder heterocyclischen Reste können mit den vorstehend genannten Substituenten einfach oder mehrfach substituiert sein, wobei auch Kombinationen der vorstehend genannten Substituenten möglich sind.

Ferner umfaßt der Begriff Arginin-Mimetika im Rahmen der vorliegenden Erfindung auch Modifikationen der N-terminalen Aminogruppe sowie der C-terminalen Carbonylgruppe, mit der Maßgabe, daß derartige Modifikationen dieselben bzw. im wesentlichen dieselben räumlichen Anordnungen aufweisen, die für das nicht modifizierte Arginin-Rückgrat typisch sind. Ein Beispiel für eine derartige Modifikation ist die Reduktion der C-terminalen Carbonylgruppe zu einer CH₂-Gruppe.

Grundsätzlich können bei der vorliegenden Erfindung auch andere aus dem Stand der Technik bekannte oder davon abgeleitete Arginin-Mimetika eingesetzt werden, mit der Maßgabe, daß das Arginin-Mimetikum den sterischen Anforderungen für ein P1-Substrat des Faktor Xₐ-Proteins entspricht und der C-terminale Rest des Arginin-Mimetikum in zwischenmolekulare Wechselwirkungen mit der S3/S4-Tasche des Faktor Xₐ-Proteins treten kann.

Als Arginin-Mimetikum wird bei der vorliegenden Erfindung ein Phenylalanin-Analogon verwendet, das am aromatischen Ring mit einem basischen Rest substituiert ist. Der basische Substituent ist am meisten bevorzugt eine Amidino-Gruppe an der 3-Position des aromatischen Rings.

Im Fall der Verwendung eines Aminosäureanalogons wie dem vorstehend beschriebenen Phenylalanin-Analogon als Arginin-Mimetikum wird der vorstehend beschriebene vorteilhafte Bindungsmodus des erfindungsgemäßen Faktor Xₐ-Inhibitors dadurch erreicht, daß die Chiralität am C°-Atom des Arginin-Mimetikums bzw. an einem entsprechenden Chiralitätszentrum eines Rückgrat-modifizierten Arginin-Mimetikums R ist, so daß der C-terminal mit dem Arginin-Mimetikum verknüpfte Rest in die S3/S4-Tasche des Faktor Xₐ-Proteins reicht und dort in zwischenmolekulare Wechselwirkungen mit der hydrophoben Gruppen der S3/S4-Tasche treten kann.

Ein (R)-chirales Aminosäureanalogon als Arginin-Mimetikum weist ferner den Vorteil auf, daß es gegenüber (S)-chiralen Aminosäureanaloga eine gesteigerte Stabilität aufweist und somit der Inhibitor bei einer pharmakologischen Anwendung im Körper länger aktiv sein kann.

Der C-terminale Rest des Arginin-Mimetikums umfaßt bei einer bevorzugten Ausführungsform der vorliegenden Erfindung einen direkt mit dem Arginin-Mimetikum verbundenen Linker sowie eine gegebenenfalls substituierte, hydrophobe Gruppe, die in zwischenmolekulare Wechselwirkungen mit der hydrophoben S3/S4-Tasche des Faktor Xₐ-Proteins treten kann.

Der Linker weist vorzugsweise eine Größe auf, die zur Überbrückung der S2-Tasche des Faktor Xₐ-Proteins geeignet ist, d.h. seine räumlichen Anordnung ist vorzugsweise ähnlich der des natürlichen P2-Substrats Gly.

Die hydrophobe Gruppe des C-terminalen Rests weist vorzugsweise eine räumliche Anordnung auf, die einen optimalen Fit der hydrophoben Gruppe in die S3/S4-Tasche des Faktor Xₐ-Proteins ermöglicht. Ferner ist es vorteilhaft, wenn die hydrophobe Gruppe mit einem oder mehreren basischen Substituenten substituiert ist, die derart angeordnet sind, daß Wechselwirkungen mit negativ geladenen bzw. negativ polarisierten Gruppen des Faktor Xₐ-Proteins in der Umgebung der S3/S4-Tasche möglich sind. Bevorzugte basische Substituenten sind Amidino-, Guanidino-, Amino-, Alkylamino-, Aminoalkyl-, Amid-Substituenten und dergleichen.

Die vorliegende Erfindung betrifft Verbindungen gemäß der folgenden Strukturformel I: 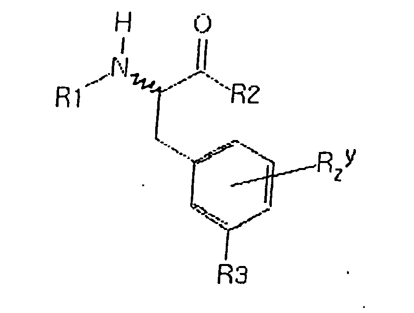 in der:
- R¹ einen direkt mit dem Phenylalaninanalogon verbundenen Linker L¹ ausgewählt aus einer Bindung, einer Gruppierung R^{x}, -CO-, -CO-NH-, -COO-, -CS-, -CS-NH-, -COS- oder -CO-CH₂-NH und eine Gruppe R⁴ umfasst, wobei R⁴ ein ein- oder mehrfach mit R^{Y} substituierter oder nicht substituierter, gesättigter oder ungesättigter carbocyclischer Rest, heterocyclischer Rest oder Alkylrest ist; R^{x} C₁- C₁₀-Alkylen, C₁-C₁₀-Alkenylen oder C₁-C₁₀-Alkinylen bedeutet, welches gegebenenfalls Heteroatome in der Kette aufweist;
- R² einen direkt mit dem Phenylalaninanalogon verbundenen Linker L² ausgewählt aus -OR⁵-, -NH-R⁵-, -NH-NH-R¹- oder -CH₂-R⁵-, wobei R⁵ ein substituierter oder nicht substituierter, gesättigter oder ungesättigter carbocyclischer, heterocyclischer oder nicht cyclischer Alkylrest oder eine Gruppe R^{x} sein kann und eine Gruppe R⁷ umfasst, wobei R⁷ ein ein- oder mehrfach mit R⁸ substituierter oder nicht substituierter C₁₋₃₀-Alkyl-, C₂₋₃₀-Alkenyl-, C₂₋₃₀-Alkinyl-, C₃₋₃₀-Cycloalkyl-, C₅₋₃₀-Aryl-, C₃₋₃₀-Heteroaryl-, C₆₋₃₀-Alkaryl- oder C₄₋₃₀-Alkheteroaryl-Rest ist und R⁸ ein Amidino-, Guanidino-, Amino-, Ester-, Alkylamino-, Aminoalkyl, Cyano-, Amid- oder Hydroxylrest oder/und Halogen, -OH, -NH₂, -Formyl, -Acetyl, -OMe, -OEt, -NHMe, -NHEt, -SH, SEt, -SMe, -NMe₂, -CH₃, -CH₂OH, -CH₂CH₃, -NHOH, -COOH, -COOMe, CN, NO₂ oder -CH₂CH₃ ist,
- R³ ein Amidino- oder ein Guanidinorest an der 3-Position des aromatischen Rings des Phenylalaninrests ist und der aromatische Ring gegebenenfalls mit zusätzlichen Substituenten R^{y}, ausgewählt aus Halogen, -OH, -NH₂, -Formyl, -Acetyl, -OMe, -OEt, -NHMe, -NHEt, -SH, SEt, SMe, -NMe₂, -CH₃, -CH₂OH, -CH₂CH₃, - NHOH, -COOH, -COOMe, CN, NO₂, -CH₂CH₃, substituiert ist,
wobei z = 0 bis 4.

Der Linker L¹ dient zur Verknüpfung der Gruppe R⁴ an das Stickstoffatom des Phenylalaninanalogons der Formel I. L¹ kann deshalb jede Gruppierung darstellen, die eine solche Verknüpfung ermöglicht. Bevorzugt stellt L¹ eine Gruppierung dar, die chemisch und enzymatisch stabil ist, um einen Abbau der Verbindung der Formel I bei einem Einsatz als pharmazeutisches Mittel zu vermeiden.

Der Linker L¹ kann einfach eine Bindung darstellen. Dann ist R⁴-L¹NH... gleich R⁴-NH... Bevorzugt umfasst der Linker L¹ eine Gruppierung R^{x} mit einer Kettenlänge von 1 bis 10 Atomen, bevorzugt 1 bis 5 Atomen, wie z.B. C₁-C₁₀-, insbesondere C₁-C₅-Alkyl, C₁-C₁₀-, insbesondere C₁-C₅-Alkenyl, C,-C₁₀-, insbesondere C₁-C₅-Alkinyl, wobei diese Gruppierung auch Heteroatome, insbesondere O, S oder N in der Kette aufweisen kann, z.B. (O-CH₂-CH₂)ₙ mit n = 1 bis 3. besonders bevorzugt umfasst der Linker L¹ zusätzlich zu der genannten Gruppierung R^{x} oder ohne die genannte Gruppierung R^{x} eine Verknüpfungsgruppe, die an dem Stickstoff als Phenylalaninanalogons gebunden ist.

Besonders bevorzugt umfasst R¹ einen zur Ausbildung von Wasserstoffbrücken fähigen Linker L¹. Zur Ausbildung von Wasserstoffbrücken fähige Linker L¹ bzw. Verknüpfungsgruppen bzw. potenzielle Wasserstoffakzeptoren bzw. -donatoren, die zudem aufgrund ihrer Geometrie bevorzugt sind, umfassen Linker wie -CO-, -CO-NH- oder -COO-, die mit der NH-Gruppe des Phenylalaninanalogons eine Amidbindung (für -CO-), eine Harnstoffbindung (für -CO-NH-) oder eine Urethanbindung (für - COO-) bilden. Ebenfalls ist eine N-terminale Verknüpfung der Gruppe R⁴ über einen -SO₂-Linker möglich.

Beispiele für bevorzugte Reste R¹ sind -CO-R⁴, -CO-NH-R⁴ oder -C-OOR⁴ und entsprechende Schwefelgruppen -CS-R⁴, -CS-NH-R⁴ oder -COSR⁴ Besonders bevorzugt ist R¹ = COOR⁴. Noch mehr bevorzugt R¹ = -CO-NH-R⁴. Zwischen der Verknüpfungsgruppe und dem Rest R⁴ kann die oben genannte Gruppierung R^{x} angeordnet sein. Es wurde festgestellt, dass Harnstoffderivate (L¹ = -CO-NH-) FXₐ hervorragend hemmen und chemisch und enzymatisch äußerst stabil sind, weshalb sie sich besonders gut als Inhibitoren für FXₐ eignen.

Bei dem Linker L¹ kann es sich aber auch um Glycin (-CO-CH₂-NH-) oder eine andere natürliche oder nicht-natürliche Aminsäure (-CO-CHR-NH-) handeln.

Die Gruppe bzw. der Rest R⁴ ist bevorzugt ein hydrophober Rest. Es kann sich aber auch um einen hydrophilen Rest oder einen Rest mit einer hydrophoben Gruppierung, welche einen oder mehrere hydrophile Substituenten trägt, handeln. R⁴ kann beispielsweise ein gesättigter oder ungesättigter, substituierter oder nicht substituierter, nicht-cyclischer -Alkylrest; ein gesättigter oder ungesättigter, substituierter oder nicht substituierter carbocyclischer Rest; oder ein gesättigter oder ungesättigter, substituierter oder nicht substituierter heterocyclischer Rest sein.

Bevorzugt ist R⁴ ein C₁₋₃₀-Alkyl-, C₂₋₃₀-Alkenyl-, C₂₋₃₀-Alkinyl-, C₃₋₃₀-Cycloalkyl-, C₅₋₃₀-Aryl-, C₃₋₃₀-Heteroaryl-, C₆-C₃₀-Alkaryl- oder C₄₋₃₀-Alkheteroaryl-Rest, wobei diese Reste einen oder mehrere Substituenten tragen können. R⁴ umfasst bevorzugt mindestens 4 C-Atome, mehr bevorzugt mindestens 6 C-Atome und bevorzugt bis zu 24 C-Atome, mehr bevorzugt bis zu 18 C-Atome. Geeignete Heteroatome, welche der Rest R⁴ enthalten kann, sind z.B. O, N, S und P. Der Rest R⁴ kann weiterhin einen oder mehrere Substituenten aufweisen. R⁴ ist bevorzugt ein mit mindestens einem Rest R⁶ substituierter, nicht cyclischer C₁- bis C₅-Alkylrest, wobei R₆ aus CₙH₂ₙ₊₁ mit n = 1 bis 10 ausgewählt ist. Besonders bevorzugt ist R⁴ t-Butyl. Bei einer weiteren bevorzugten Ausführungsform ist R⁴ substituiertes oder nicht substituiertes Phenyl, Benzyl, Fluorenyl, Naphtyl, -C(CH₃)₂-C₆H₅ oder Adamantyl. Am meisten bevorzugt ist R⁴ = Adamantyl.

Besonders bevorzugt sind weiterhin Reste R⁴, die größer (vom eingenommenen Volumen) als der Phenylrest sind.

Der direkt mit dem Phenylalaninanalogon verbundene Linker L² weist erfindungsgemäß bevorzugt eine Größe auf, die zur Überbrückung der S2-Tasche des Faktor Xₐ-Proteins geeignet ist, d.h. seine räumliche Anordnung ist vorzugsweise ähnlich der des natürlichen P2-Substrats Gly. Dabei ist der Linke von R² vorzugsweise -OR⁵-, -NH-R⁵-, -NH-NH-R⁵- oder -CH₂R⁵, wobei R⁵ ein substituierter oder nicht substituierter, gesättigter oder ungesättigter carbocyclischer, heterocyclischer oder nicht cyclischer Alkylrest ist oder eine wie oben definierte Gruppe R^{x} sein kann. Besonders bevorzugt ist der Linker L² = -NH-R⁵-. Der Linker L² kann aber auch einfach eine Bindung sein.

Vorteilhaft für einen erfindungsgemäßen Faktor Xₐ-Inhibitor - mit der Strukturformel 1 ist, wenn ein hydrophober Rest R⁵ über eine Ester- oder Amidbindung C-terminal mit dem Phenylalaninderivat verknüpft ist, wobei R⁵ besonders bevorzugt ein substituierter oder nicht substituierter C₁- bis C₅-Alkylrest ist. Dabei kann R⁵ die Formel -(CH₂)ₘ aufweisen, in der m = 1 bis 3 ist. Besonders bevorzugt ist R⁵= -CH₂-CH₂-. Ferner umfasst R² eine nicht substituierte oder mit einem oder mehreren Resten R⁸ substituierte, gesättigte oder ungesättigte Gruppe R⁷, die ein nicht cyclischer Rest sein kann aber insbesondere ein carbocyclischer Rest wie ein cyclischer Alkyl-, Alkylaryl-, Arylalkyl- oder Arylrest oder ein mindestens ein Heteroatom wie beispielsweise Sauerstoff, Stickstoff und/oder Schwefel enthaltender heterocyclischer Rest ist, wobei R⁸ vorzugsweise ein basischer und/oder ein als Wasserstoffbrücken-Donator oder -Akzeptor fungierender Substituent und/oder ein Halogen ist.

Bevorzugt ist R⁷ ein C₁₋₃₀-Alkyl-, C₂₋₃₀-Alkenyl-, C₂₋₃₀-Alkinyl-, C₃₋₃₀-Cycloalkyl-, C₅₋₃₀-Aryl-, C₃₋₃₀-Heteroaryl-, C₆-C₃₀-Alkaryl- oder C₄₋₃₀-Alkheteroaryl-Rest, wobei diese Reste einen oder mehrere Substituenten tragen können. R⁷ umfasst bevorzugt mindestens 4 C-Atome, mehr bevorzugt mindestens 6 C-Atome und bevorzugt bis zu 24 C-Atome, mehr bevorzugt bis zu 18 C-Atome. Geeignete Heteroatome, welche der Rest R⁷ enthalten kann, sind z.B. O, N, S und P. Der Rest R⁷ kann weiterhin einen oder mehrere Substituenten aufweisen.

Insbesondere kann R⁷ ein nicht substituierter oder mit einem oder mehreren Resten R⁸ substituierter Phenyl-, Piperidin-, Pyrrol-, Furan-, Thiophen-, Pyridin-, Naphthalin-, Antracen- oder Indolrest sein. Andere aromatische, auch kondensierte aromatische, oder heteroaromatische Reste sind ebenfalls denkbar.

R⁸ ist bevorzugt ein Rest, der unter physiologischen Bedingungen, z.B. einem pH-Wert von ca. 6,5 - 7,5 ein positiv geladener Rest ist.

Besonders bevorzugt ist R⁸ ein Amidino-, Guanidino-, Amino-, Ester-, Alkylamino-, Aminoalkyl-, Cyano-, Amid- oder Hydroxylrest o.dgl.

Besonders vorteilhaft für die Verwendung der erfindungsgemäßen Verbindungen mit der Strukturformel I als Faktor Xₐ-Inhibitor kann als Rest R² ein synthetisch auf einfache Weise durch Veresterung einer natürlichen oder nicht natürlichen Aminosäure erzeugbarer Rest -NH-CHR⁹-COO-(CH₂)ₘR⁷ eingesetzt werden, bei dem m = 1 bis 5 ist, R⁷ wie vorstehend definiert ist und R⁹ eine derivatisierte oder nicht derivatisierte Seitenkette einer natürlichen Aminosäure ist.

R³ ist bei der vorliegenden Erfindung ein Amidino- oder ein Guanidino-Rest, besonders bevorzugt ein Amidinorest. Der aromatische Ring des Phenylalaninrests ist an der 3-Position durch den Rest R³, z.B. einem Amidinorest, substituiert.

Ferner kann der Ring auch vorteilhaft zusätzlich mit einem oder mehreren Substituenten R^{Y} substituiert sein, wobei z = 0 bis 4. Bevorzugt sind polare Substituenten, wie Halogen, z.B. Fluor, Chlor, Brom, Jod, Hydroxyl oder Alkoxy und/oder basische Substituenten.

R^{y} kann bevorzugt jeweils unabhängig bei jedem Auftreten ein Halogen, z.B. Fluor, Chlor, Brom, Jod, -OH, -NH₂, -Formyl, -Acetyl, -OMe (Me = Methyl), - OEt (Et = Ethyl), NHMe, -NHEt. SH, SEt, SMe, NMe₂, -CH₃, -CH₂OH, -CH₂-CH₃, -NH-OH, -COOH, -COOMe, CN, NO₂, -CH₂-CH₃ bedeuten.

Die für den Substituenten R^{Y} angegebenen Gruppierungen stellen auch bevorzugte Substituenten für die übrigen hierin angegebenen substituierten Gruppen (z.B. bei R⁴, R⁵, R⁷) dar, soweit nicht explizit anders angegeben.

Überraschenderweise wurde gefunden, dass es für die Inhibierung des Faktor Xₐ mit einer erfindungsgemäßen Verbindung der Strukturformel (1) vorteilhaft ist, wenn das Phenyl-alaninanalogon (R)-chiral ist, weil dann der C-terminale Rest in zwischenmolekulare Wechselwirkungen mit der S3/S4-Tasche des Faktors Xₐ-Proteins treten kann. Bevorzugt sind deshalb die oben genannten Verbindungen in der (R)-Konformation. Die Erfindung umfasst aber auch die Verbindungen in (S)-Konformation sowie Gemische aus (R)- und (S)-Enantiomeren.

Eine sehr starke Inhibitorwirkung für Faktor Xₐ wurde durch die erfindungsgemäße Verbindung N-1-Adamantyloxycarbonyl-D-3-amidinophenylalanin-(2-phenyl)-1-ethylamid erreicht.

Eine noch stärkere Inhibitorwirkung für Faktor Xₐ wurde für die erfindungsgemäße Verbindung N-(1-Adamantylaminocarbonyl)-D-3-amidinophenylalanin-(2-phenyl)-1-ethylamid beobachtet.

Die erfindungsgemäße Verbindung kann in freier Form oder als pharmazeutisch akzeptables Salz, wie beispielsweise als Hydrochlorid, vorliegen.

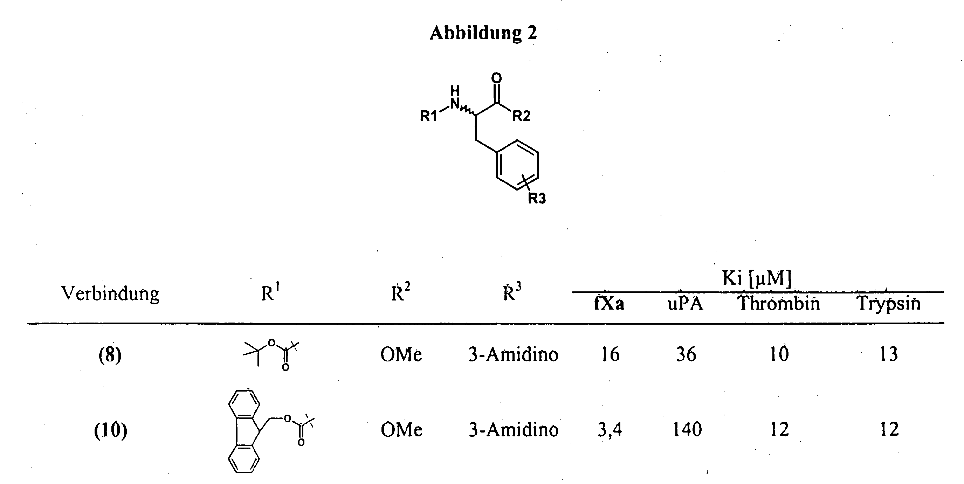

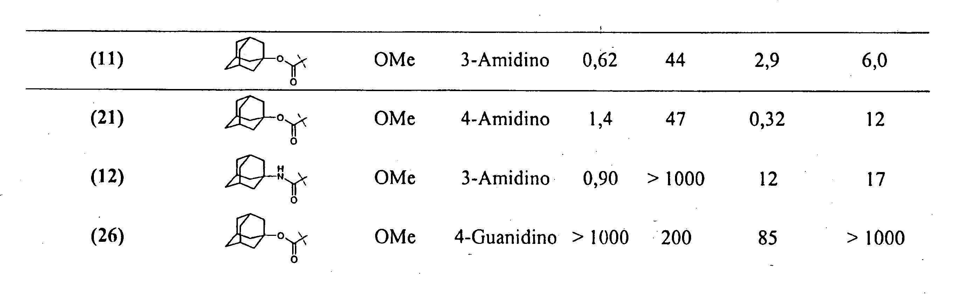

Im folgenden wird die vorliegende Erfindung durch repräsentative erfindungsgemäße Verbindungen veranschaulicht. In den Abbildungen 2 bis 4 sind deren Inhibitorstärken gegenüber Faktor Xₐ und zum Vergleich gegenober uPA. Thrombin und Trypsin aufgeführt.

Die Sytnhese und Inhibierungskonstanten der Verbindungen 8. 10-12, 21 und 26-28 stellen keine erfindungsgemäßen Verbindungen dar, sondern dienen lediglich als Vorgleichsbeispiele.

Die Präferenz von Faktor Xₐ für 3-Amidino- gegenüber 4-Amidinophenylataninderivaten stimmt mit vorangehenden Veröffentlichungen überein (Maduskuie et al, vide supra), obwohl bei der inversen Bindung der Bisbenzamidinverbindung **2** eine Präferenz für die 4-Amidinogruppe beobachtet wurde (B. Gabriel et al., vide supra). Interessanterweise erfüllt das 4-Guanidinoderivat **26** keineswegs die sterischen Erfordernisse für einen P1-Rest für dieses Enzym, da Im Vergleich mit Verbindung **19** ein dramatischer Verlust der inhibitorischen Aktivität beobachtet wird. Dies gilt ebenso für die anderen untersuchten Enzyme uPA, Thrombin und Trypsin.

Der Vergleich der racemischen Verbindung **15** als freie Säure am C-Terminus mit der racemischen Verbindung **17** als C-terminales Amidderivat zeigt keine bedeutenden Unterschiede. was mit der Kristallstruktur von mit des-Gla-Faktor Xₐ komplexiertem DX-9065a übereinstimmt, bei der die freie Carboxylatgruppe in das umgebende Lösungsmittel reicht (H. Brandstetter et al. (1996), J. Biol. Chem. 271, 29988-29992). Auf ähnliche Weise wurde im Stand der Technik beobachtet, daß die Faktor Xₐ-inhibierung nicht beeinflußt wird, wenn die Verbindung **2** anstelle eines freien Carboxyls als Esterderivat vorliegt (Gabriel et al., vide supra). Angesichts dieser Ergebnisse ist die signifikant verstärkte inhibitorwirkung der Verbindung **11,** dem C-terminalen Methylesterderivat, verglichen mit der Verbindung **15** mit der freien C-terminalen Carboxylgruppe äußerst überraschend. Offensichtlich unterscheidet sich die Art der Bindung von der des Daiichi-Inhibitors DX-9065a (Brandstetter et al. vide supra) oder von der der Verbindung **1** (M. Renatus et al. (1998), J. Med. Chem. 41, 5445 bis 5456), indem die Estergruppe in eine neuen Art von Wechselwirkung in der Nähe der S1-Bindungsstelle involviert ist.

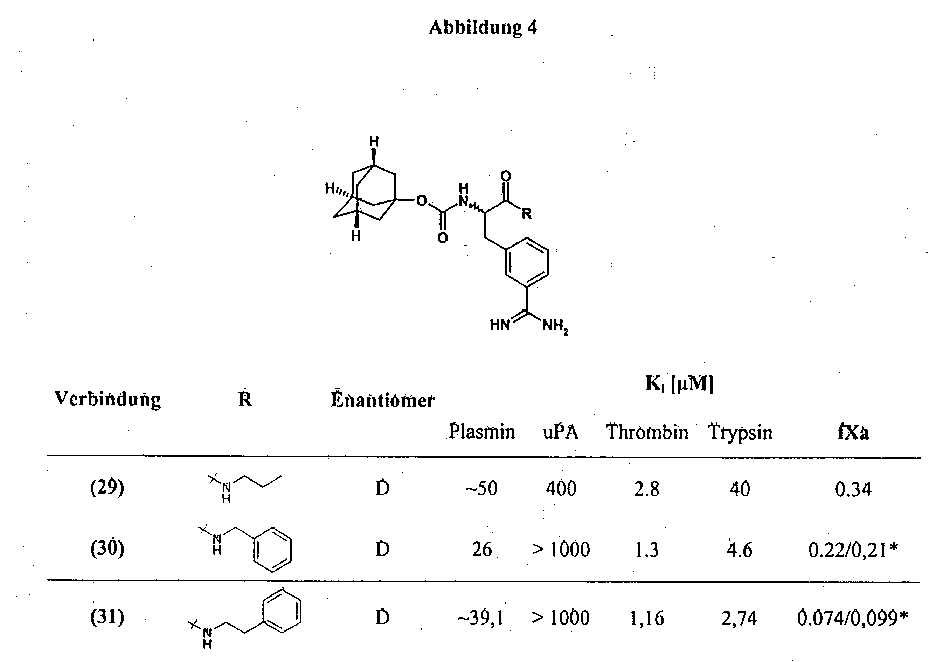

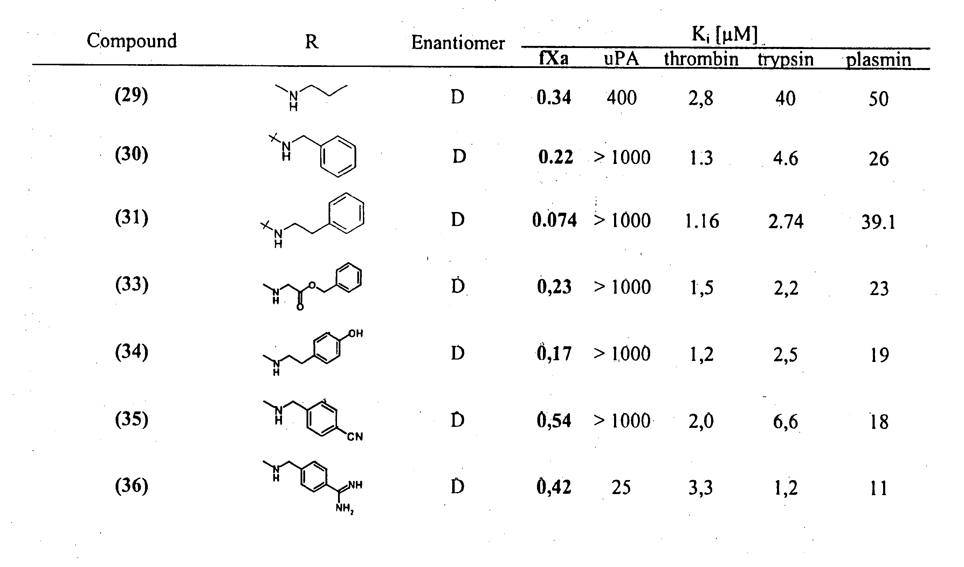

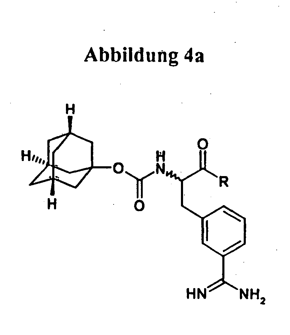

Der im vorstehenden Absatz beschriebene vorteilhafte Effekt auf die Inhibitorwirkung wird verstärkt, wenn beispielsweise ein C-terminaler Ester oder ein Gterminales Amidderivat verwendet wird, das eine hydrophobe Gruppe sowie einen Linker umfaßt, welcher eine zur Überbrückung der S2-Tasche des Faktor Xₐ-Proteins geeignete Größe aufweist, d.h. dessen räumlichen Anordnung ähnlich der des natürlichen P2-Substrats Gly ist. Dies wird durch die erfindungsgemäßen Verbindungen **29** bis **31** veranschaulicht (siehe Abbildung 4). So entspricht bei dem stärksten Inhibitor 31 die Gruppe -NH-CH₂-CH₂- dem vorstehend beschriehenen linker, indem er nahezu dieselbe räumliche Ausdehnung wie ein Gly-Rest -NH-CH₂-CO- aufweist. Als hydrophobe Gruppe ist eine wie vorstehend beschriebene substituierte oder nicht substituierte Aryl- oder Alkylarylgruppe bevorzugt, um optimale zwischenmolekulare Wechselwirkungen mit der hydrophoben S3/S4-Tasche des Faktor Xₐ-Proteins und damit eine starke Inhibitorwirkung zu erreichen.

Am Beispiel der racemischen Verbindung **11,** wird der Effekt der Chiralität des 3-Amidinaphanylalaninderivats auf die Faktor Xₐ-Inhibierung dargestellt-Die Kᵢ-Werte des L-(Verbindung **27)** bzw. des D-Enantiomers (Verbindung **28)** sind in Tabelle 3 aufgeführt. Durch Kristallstrukturanalyse des bekannten Trypsin/**11**-Komplexes sowie durch auf dieser Kristallstruktur basierenden Modelling-Studien für Trypsin und Faktor Xₐ kann keine klare Präferenz zwischen dem L-(Verbindung **27)** und D-Enantiomer (Verbindung **28)** ermittelt werden. Dies wird durch die Kᵢ-Werte von Trypsin bestätigt, da beide Enantiomere **27** und **28** durch Trypsin mit beinahe identischer Affinität nur mit einer geringen Bevorzugung für das D-Enantiomer erkannt werden. Dagegen zeigt das D-Enantiomer **28** bei der Inbibierung von Faktor Xₐ mit einem Kᵢ = 0,39 µM überraschenderweise eine etwa 10 mal größere Aktivität als das L-Enantiomer **27.** Während die Affinität dieses Typs von inhibitor für Trypsin und Thrombin nur marginal durch die Chiralität des 3-Amidinophenylalaninrestes beeinflußt wird, ist dagegen uPA nur zur Erkennung des L-Enantiomers fähig. Damit wird erstmals eine Abhängigkeit der Inhibitorstärke für Faktor Xₐ von der Chiralität des verwendeten Arginin-Mimetikums und insbesondere erstmals ein R-chirales Argininmimetikum als wirksamer Faktor Xₐ-Inhibitor beschrieben.

Diese Präferenz von Faktor Xₐ für die (R)-Chiralität des Arginin-Mimetikums ist ein wesentliches Merkmal der vorliegenden Erfindung und konnte aufgrund der bekannten Untersuchungen nicht erwartet werden. Ebenso überraschend ist die Beeinflussung der Selektivität hinsichtlich der Inhibierung von uPA, Trypsin und Thrombin durch die Chiralität an dieser Position, da uPA selektiv für die (S)-Chiralität ist, während sowohl Trypsin als auch Thrombin mit vergleichbaren Affinitäten beide Isomere erkennen.

Durch Modelling-Studien des Komplexes aus Faktor Xₐ und Verbindung **11** basierend auf der bekannten Kristallstruktur von Faktor Xₐ (K. Padmanabhan et al. (1993), J. Mol. Biol. 232, 947-966) kann folgende Art der Bindung vermutet werden: Die Benzamidinogruppe von N-1-Adamantyloxycarbonyl-D-3-Amidinophenylalaninmethylester **(28)** liegt in der S1-Tasche, während die Adamantylgruppe in einer leichten Vertiefung umgeben von den Seitenketten von Trp 215, Glu 217 und Phe 174 südlich der Substrat S3/S4-Arylbindungsstelle positioniert ist. Bei dieser Art der Bindung zeigt die C-terminale Estergruppe in Richtung der S3/S4-Substratbindungstasche, wodurch die Präferenz der (R)-Chiralität erklärt werden kann. Ein ähnlicher Bindungsmechanismus kann für die besonders bevorzugten erfindungsgemäßen Verbindungen **29** bis **31** vermutet werden.

Ein Vergleich von hydrophoben Kopfgruppen, die mit dem 3-Amidinophenylalamin N-terminal verbunden sind, wie beispielsweise der tert-Butylgruppe (Verbindung **8),** der 9-Fluorenylmethylgruppe (Verbindung **10),** der 1-Adamantylgruppe (Verbindung **11)** und der Benzylgruppe (Verbindunng **4),** zeigt deutlich, daß nicht-planare und nicht-aromatische Gruppen am besten geeignet sind. So führt die Verbindung **11** mit der 1-Adamantylgruppe zu einer submikromolaren Inhibierung von Faktor Xₐ und gleichzeitig zu einer bemerkenswerten Selektivität gegenüber uPA, Thrombin und Trypsin.

Der Ersatz der N-terminalen Urethangruppe als potentiellem Wasserbrückenakzeptor in Verbindung **11** durch die verwandte Harnstoffgruppe (Verbindung **12)** beeinflußt mit Ausnahme von dramatischen Effekten auf die Inhibierung von uPA offensichtlich nicht das Wasserstoffbindungsnetzwerk in diesem Abschnitt des Protease/Inhibitor-Komplexes, führt allerdings zu einer gewünschten Stabilität gegenüber Säuren, beispielsweise Magensäuren.

Hinsichtlich der Selektivität werden die deutlichsten Effekte durch eine freie Carboxylgruppe am C-Terminus erreicht, die offensichtlich auf spezifische Weise die Wechselwirkungen mit uPA, Thrombin und Trypsin an deren aktiven Stellen beeinträchtigt. Auf ähnliche Weise beeinträchtigt eine 4-Guanidinogruppe die Inhibierung nicht nur von Faktor Xₐ, sondern ebenfalls von den anderen untersuchten Trypsin-artigen Enzymen.

Die erfindungsgemäßen Verbindungen werden durch ein Verfahren synthetisiert, das die folgenden Schritte umfaßt:
a) Zugabe von R⁴-NCO, R⁴-NCS, X-CO-R⁴, X-SO₂-R⁴, X-CO-NH-R⁴ oder X-COOR⁴ zu D- oder L-Phenylalanin, das an der 3-Position den basischen Substituenten R³ oder eine Vorstufe von R³ aufweist;
b) gegebenenfalls Umwandlung der Vorstufe von R³ in den Substituenten R³;
c) gegebenenfalls Zugabe von YR⁵ zu dem Reaktionsprodukt des Schritts b). Dabei kann X = Cl oder Aktivester sein. Ebenso können die vorstehend genannten Verbindungen, die den Rest R⁴ enthalten, falls möglich, in Form ihres jeweiligen Säureanhydrids zugegeben werden.

Die N-Derivate des racemischen 3- und 4- Amidinophenylalanins werden aus den jeweiligen 3-und 4-Cyanoverbindungen, gefolgt durch deren Umwandlung zu den verwandten Amidinoderivaten, oder durch direkte Derivatisierung des Amidinophenylalanins erhalten. Aufgrund von Nebenreaktionen, die durch die ungeschützte Amidinogruppe entstehen, sowie aufgrund von Schwierigkeiten bei der Reinigung der hydrophilen Amidinoverbindungen sind Reaktionssequenzen mit einer abschließenden Erzeugung der Amidinofunktion bevorzugt. Für die Umwandlung der Cyanophenylalaninderivate in die entsprechenden Amidinoverbindungen kann die Cyanogruppe durch Zugabe von Hydroxylaminhydrochlorid und anschließende katalytische Hydrierung in den Amidinorest umgesetzt werden. Jedoch sind noch weitere Abwandlungen der zweistufigen Reaktion für die Synthese von N-Benzyloxycarbonylamidinophenylalaninpiperidid möglich, die durch Stüber et al. berichtet wurde (Stüber et al. (1998), Peptide Res. 8, 78-85).

Die C-Derivate des racemischen 3- und 4- Amidinophenylalanins können im Fall von R² = OR⁵ durch Zugabe des entsprechenden Alkohols, gegebenenfalls in Gegenwart von Säure oder DCC (Dicyclohexylcarbodiimid) erhalten werden. Im Fall von R² = NHR⁵ kann das entsprechende Amin oder eine entsprechende Aminosäure, gegebenenfalls in Gegenwart von üblicherweise in der Peptidsynthese verwendeten Kondensationsreagenzien verwendet werden. Derartige Kondensationsreagenzien sind beispielsweise HOBT und TBTU.
Ein wesentlicher Aspekt der vorliegenden Erfindung ist die Verwendung der erfindungsgemäßen Verbindungen zur Herstellung eines Mittels zur antikoagulatorischen Therapie. Unter antikoagulatorischer Therapie versteht man im Zusammenhang mit der vorliegenden Erfindung die Behandlung von Gefäßerkrankungen, um thrombotische Gefäßverschlüsse zu vermeiden (antithrombotische Therapie). Derartige Therapien umfassen die Prophylaxe und Therapie venöser Thrombosen und Lungenembolien sowie die antithrombotische Therapie arterieller Thrombosen und Embolien, einschließlich koronarer Herzkrankheiten wie Angina pectoris oder akuter Myokardininfarkt, cerebrovaskulärer Durchblutungsstörungen wie transienter ischämischer Attacken und Hirninfarkte, und peripherer arterieller Verschlußkrankheiten. Des weiteren können die erfindungsgemäßen Verbindungen zur hämorheologischen Therapie, d.h. zur Verbesserung der Fließfähigkeit des Blutes verwendet werden.

Die erfindungsgemäßen Verbindungen der Strukturformel 1 sind ebenfalls als geeignete Inibitoren von anderen Serinproteasen, insbesondere von humanem Thrombin, Plasmakallikrein und Plasmin denkbar. Bei einer solchen inhibitorischen Wirkung können die erfindungsgemäßen Verbindungen für die Verhinderung oder Behandlung von physiologischen Reaktionen, der Blutgerinnung und Entzündungsprozessen verwendet werden, die durch die vorstehend genannte Klasse von Enzymen katalysiert werden.

Ein weiterer Gegenstand der vorliegenden Erfindung ist eine pharmazeutische Zusammensetzung, die gegebenenfalls einen pharmazeutisch akzeptablen Träger und mindestens eine der erfindungsgemäßen Verbindungen enthält. Bevorzugt enthält die pharmazeutische Zusammensetzung eine therapeutisch wirksame Menge der erfindungsgemäßen Verbindungen. Unter einer "therapeutisch wirksamen Menge" wird eine Menge der erfindungsgemäßen Verbindungen mit der Strukturformel I verstanden, die bei der Verabreichung alleine oder in Kombination mit einem zusätzlichen therapeutischen Mittel an einen Säuger eine therapeutische Wirksamkeit zeigt und insbesondere antithrombotisch oder als Antitumormittel wirksam ist.

"Verabreichung in Kombination" oder "Kombinationstherapie" bedeutet im Rahmen der vorliegenden Erfindung, daß die erfindungsgemäßen Verbindungen der Formel 1 und ein oder mehrere zusätzliche therapeutische Mittel dem zu behandelnden Säuger nebeneinander verabreicht werden. Bei Zugabe in Kombination kann jede Komponente zur selben Zeit oder nacheinander in irgendeiner Reihenfolge zu verschiedenen Zeitpunkten verabreicht werden. Somit kann jede Komponente getrennt verabreicht werden, aber zeitlich ausreichend nahe beieinander, damit der gewünschte therapeutische Effekt bereitgestellt wird. Weitere Antikoagulationsmittel (oder Koagulationsinhibierungsmittel) die in Kombination mit den erfindungsgemäßen Verbindungen verwendet werden können, schließen Warfarin und Heparin sowie weitere Faktor Xₐ-Inhibitoren ein, die im Stand der Technik beschrieben worden sind.

Die Verabreichung der erfindungsgemäßen Verbindungen in Kombination mit derartigen zusätzlichen therapeutischen Mitteln kann einen Vorteil über die jeweilige Verwendung der Verbindungen und Mittel alleine bereitstellen, indem beispielsweise die Verwendung von jeweils niedrigeren Dosen möglich ist, durch welche mögliche Nebenwirkungen minimiert werden.

Die erfindungsgemäßen Verbindungen sind insbesondere zur Behandlung oder prophylaktischen Anwendung bei Krankheiten geeignet, die mit einer pathologischen Expression oder Überexpression von Faktor Xₐ assoziiert sind oder/und eine verstärkte Faktor Xₐ proteolytische Aktivität beinhalten, welche wiederum für tumorwachstumsfördernde und metastasierungsfördernde Fibrinablagerungen verantwortlich sein kann.

So sind die erfindungsgemäßen Verbindungen in der Lage, effizient das Wachstum und/oder die Ausbreitung von malignen Tumoren sowie die Metastasierung von Tumoren zu hemmen oder/und zu verhindern. Ein weiterer Gegenstand der Erfindung ist deshalb die Verwendung der erfindungsgemäßen Verbindungen zur Herstellung eines Antitumormittels. Die erfindungsgemäßen Faktor Xₐ-Inhibitoren können dabei gegebenenfalls zusammen mit geeigneten pharmazeutischen Hilfs- oder Trägerstoffen zur Herstellung von Arzneimitteln formuliert werden. Weiterhin ist es möglich, die Faktor Xₐ-Inhibitoren gegebenenfalls zusammen mit anderen Tumormitteln, anderen Wirkstoffen oder mit anderen Behandlungsarten, z.B. in Kombination mit Bestrahlung oder chirurgischen Eingriffen einzusetzen. Zur Behandlung mit den erfindungsgemäßen Verbindungen geeignete Tumore, die eine Faktor Xₐ-Aktivität aufweisen, sind insbesondere Lungen-, Blasen-, Leber- und Ovarialkarzinome sowie maligne Melanome und Neuroblastome.

Die erfindungsgemäßen Verbindungen hemmen FXa bereits in niedrigen Konzentrationen. Die hierin vorgestellte Verbindung 31 beispielsweise mit einer Inhibitionskonstante Ki = 0,074 *µ*M. Während die erwünschte FXa-Hemmung bereits bei solchen niedrigen Konzentrationen erfolgt, wird die Blutgerinnung (gemäß dem APPT-Test) hingegen erst bei wesentlich höheren Konzentrationen der erfindungsgemäßen Verbindungen beeinflusst. Dadurch können die erfindungsgemäßen Verbindungen selektiv zur Inhibierung von FXa eingesetzt werden, ohne dass gleichzeitig die Blutgerinnung beeinflusst wird. Auf diese Weise ist es möglich, die erfindungsgemäßen Verbindungen zur Krebsbekämpfung (die mit der Hemmung von FXa in Zusammenhang steht) einzusetzen, während Nebenwirkungen, wie etwa Blutungen (die mit der Blutgerinnung zusammenhängen) vermieden werden können. Dies stellt einen wesentlichen Vorteil der erfindungsgemäßen Verbindungen gegenüber anderen fXa-Inhibitoren, wie etwa dem bekannten DX-9065a dar (Kakkar et al., J. Clinical Pathology - Clinical Molecular Pathology Edition 48(5):M28B-M290, 1995; Gouinthibault et al., British Journal of Haematology 90 (3): 689-680; Nakata et al., Cancer Letters 122 (1-2): 127-133, 1998; Yoshida et al., Fibrinolysis & Proteolysis, 11 (3):147-154, 1997; Barendszjanson et al., Tumor Biology, 19(2): 104-112, 1998; Donnelly et al., Thrombosis & Haemostasis, 79 (5): 1041-1047, 1998; Fielding et al., Blood, 91(5): 1802-1809, 1998; Tanabe et al., Thrombosis Research, 96(2): 135-143, 1999).

Die pharmazeutische Zusammensetzung kann auf alle bekannten Arten verabreicht werden, bei Menschen und Tieren beispielsweise topisch, oral, rektal oder parenteral, z.B. subkutan oder intravenös. Darüber hinaus kann sie auch in Form von Tabletten, Dragees, Kapseln, Pellets, Supositorien, Lösungen oder transdermalen Systemen, wie etwa Pflastern, verabreicht werden.

Die erfindungsgemäßen Verbindungen sind ebenfalls nützlich als Standard- oder Referenzverbindungen, beispielsweise als Qualitätsstandard oder Kontrolle in Tests oder Assays, die die Inhibierung des Faktors Xₐ einschließen. Derartige Verbindungen können in einem kommerziellen Kit, beispielsweise zur Verwendung in der den Faktor Xₐ umfassenden pharmazeutischen Forschung bereitgestellt werden.

Die erfindungsgemäßen Verbindungen können ebenfalls in diagnostischen Assays verwendet werden, die den Faktor Xₐ einschließen.

Die erfindungsgemäßen Verbindungen können in oralen Dosierungsformen wie Tabletten, Kapseln, Pillen, Pulvern, Granulen, Elixieren, Tinkturen, Suspensionen, Sirupen und Emulsionen verabreicht werden. Sie können ebenfalls intravenös, intraperitoneal, subcutan oder intramuskulär verabreicht werden, jeweils unter Verwendung von Dosierungsformen, die dem Fachmann wohl bekannt sind. Sie können alleine verabreicht werden, werden aber vorzugsweise mit einem pharmazeutischen Träger verabreicht, der auf der Basis des ausgewählten Wegs der Verabreichung und üblichen pharmazeutischen Vorgehensweisen ausgewählt wird.

Die Dosis der erfindungsgemäßen Verbindungen wird selbstverständlich von verschiedenen bekannten Faktoren, wie den pharmakodynamischen Charakteristika des speziellen Mittels und seiner Art und dem Verabreichungsweg; der Spezies, dem Alter, dem Geschtecht, der Gesundheit, der medizinischen Bedingung und dem Gewicht des Rezipienten und weiteren bekannten Faktoren abhängen. Ein Fachmann ist ohne weitere Anleitung in der Lage, die wirksame Menge der erfindungsgemäßen Verbindungen zur Herstellung eines Mittels zur antithrombotischen Therapie zu bestimmen.

Im allgemeinen wird die tägliche orale Dosis der jeweiligen aktiven Bestandteile, bei Verwendung für die vorstehend genannten Effekte, im Bereich von etwa 0,001 bis 1000 mg/kg Körpergewicht, vorzugsweise von etwa 0,01 bis 100 mg/kg Körpergewicht pro Tag und am meisten bevorzugt von etwa 1,0 bis 20 mg/kg pro Tag liegen. Intravenös liegen die am meisten bevorzugten Dosen in einem Bereich von etwa 1 bis etwa 10 mg/kg/min während einer Infusion mit konstanter Rate. Die erfindungsgemäßen Verbindungen können in einer einzelnen täglichen Dosis oder aufgeteilt auf Dosen von 2, 3 oder 4 Einnahmen täglich verabreicht werden.

Die erfindungsgemäßen Verbindungen können ebenfalls in intranasaler Form oder auf transdermalem Weg verabreicht werden.

Die erfindungsgemäßen Verbindungen werden typischerweise in Mischung mit geeigneten pharmazeutischen Verdünnungsmitteln, Exzipienten oder Trägern (die gemeinsam im folgenden als pharmazeutische Träger bezeichnet sind) hinsichtlich der beabsichtigten Form der Verabreichung und übereinstimmend mit herkömmlichen pharmazeutischen Vorgehensweisen geeignet ausgewählt werden.
Beispielsweise kann bei oraler Verabreichung in Form einer Tablette oder Kapsel die aktive Wirkstoffkomponente in Form der erfindungsgemäßen Verbindung mit einem oralen, nicht toxischen, pharmazeutisch akzeptabien, inerten Träger wie Lactose, Stärke, Sucroce, Glucose, Methylcellulose, Magnesiumstearat, Dicalciumphosphat, Calciumsulfat, Mannitol, Sorbitol und dergleichen kombiniert werden. Für die orale Verabreichung in flüssiger Form können orale Wirkstoffkomponenten mit irgendeinem oralen, nicht toxischen, pharmazeutisch akzeptablen inerten Träger wie Ethanol, Glyzerin, Wasser und dergleichen kombiniert werden.

Ferner können, falls notwendig oder gewünscht, geeignete Bindemittel, Schmiermittel, Sprengmittel und Färbemittel ebenfalls in der pharmazeutischen Zusammensetzung verwendet werden. Geeignete Bindemittel schließen Stärke, Gelatine, natürliche Zucker wie Glucose oder beta-Lactose, natürliche und synthetische Kautschuke, Carboxymethylcellulose, Polyethylenglycol, Wachse und dergleichen ein. Bei diesen Dosisformen verwendete Schmiermittel schließen Natriumoleat, Natriumstearat, Magnesiumstearat, Natriumbenzoat, Natriumacetat, Natriumchlorid und dergleichen ein. Die Sprengmittel umfassen unter anderem Stärke, Methylcellulose, Agar, Bentonit und dergleichen.

Die erfindungsgemäßen Verbindungen können ebenfalls in Form von liposomalen Transportsystemen verabreicht werden, Liposomen können aus einer Vielzahl von Phospholipiden wie Cholesterol, Stearylamin oder Phosphatidylcholinen gebildet werden.

Die erfindungsgemäßen Verbindungen können ebenfalls mit löslichen Polymeren als Wirkstoffträgern verbunden werden. Derartige Polymere schließen Polyvinylpyrrolidon, Pyrancopotymer, Polyhydroxypropytmethacrylamidphenol, Polyhydroxyethylaspartamidphenol oder mit Palmitoylresten substituiertes Polyethylenoxidpolylysin ein. Des weiteren können die erfindungsgemäßen Verbindungen mit einer Reihe von bioabbaubaren Polymeren gekoppelt werden, die nützlich zum Erreichen einer gesteuerten Freigabe eines Wirkstoffes sind, wie beispielsweise mit Polyglykolsäure, Polymilchsäure, Copolymere von Polyglykolsäure und Polymilchsäure, Polyepsiloncaprolacton, Potyhydroxy-butyrsäure. Polyorthoestern, Polyacetalen und dergleichen.

Lipiddosierungsformen für die orale Verabreichung können Färbe- und Geschmacksmittel zur Steigerung der Akzeptanz beim Patienten enthalten.

Im allgemeinen sind Wasser, ein geeignetes Öl, Salzlösungen, wäßrige Dextrose (Glukose) und verwandte Zuckerlösungen und Glykole wie Propylenglykol oder Polyethylenglykole geeignete Träger für parenterale Lösungen. Lösungen für die parenterale Verabreichung enthalten vorzugsweise ein wasserlösliches Salz des aktiven Bestandteils, geeignete Stabilisierungsmittel und, falls notwendig, Puffersubstanzen. Antioxidierende Mittel wie Natriumdisulfit, Natriumsulfit oder Ascorbinsäure, entweder alleine oder in Kombination, sind geeignete Stabilisierungsmittel.

Weitere geeignete pharmazeutische Träger sind in "Remingtons Pharmaceutical Sciences", Mack Publishing Company, einem Standardreferenzwerk auf diesem Gebiet beschrieben.

Die erfindungsgemäßen Verbindungen können auch als Lead-Substanzen eingesetzt werden. Sie können insbesondere dazu herangezogen werden, um weitere effektive Faktor Xₐ-Inhibitoren zu entwickeln oder aufzufinden, beispielsweise unter Verwendung von entsprechenden gegebenenfalls computergestützten Algorithmen. Die erfindungsgemäßen Verbindungen als Leäd-Substanzen können insbesondere zur Entwicklung von neuen Antithrombotischen und Antitumormitteln verwendet werden.

Die folgenden Beispiele dienen der Erläuterung der Erfindung, ohne diese in irgendeiner Weise einzuschränken.

### Beispiele

Sämtliche Lösungsmittel und Reagenzien, die bei den folgenden Beispielen verwendet wurden, waren von der höchsten kommerziell erhältlichen Qualität und wurden, falls erforderlich, durch Standardverfahren weiter gereinigt und getrocknet. Analytische HPLC wurde an ET 125/4 Nucleosil 100/C₈-Säulen (Macherey-Nagel, Düren, Deutschland) unter Verwendung eines linearen Gradienten von MeCN/2% H₃PO₄ von 5:95 (A) bis 80:20 (8) in 12 Minuten durchgeführt. ESI-MS Spektren wurden an einem Perkin Elmer API 165-Massenspektrometer aufgenommen (Perkin Elmer, Langen, Deutschland). TLC wurde auf Silicagel-60-Platten unter Verwendung der folgenden Lösungsmittelsysteme durchgeführt: (A) CHCl₃/MeOH/AcOH, 40:10:2; (B) CHCl₃/MeOH/AcOH, 20:20:1; (C) AcOEt/*n*-BuOH/H₂O/AcOH, 10:6:2:2 ; (D) CHCl₃/MeOH/AcOH, 190:10:2; (E) CHCl₃/MeOH/NH₃, 20:20:9; (F) CHCl₃/MeOH/AcOH, 10:20:1; (G)*n*-Hexan/AcO:Et/AcOH 49:49:2.

Die Synthese und Inhibierungskonstanten der Verbindungen 1 und 4 sind dem Stand der Technik entnommen (B. Gabriel (1998), Doktorarbeit, Technische Universität München). N,N'-Dibenzyloxycarbonyl-N"-trifylguanidin wurde nach Feichtinger et al. (J. Org. Chem. 63, 3804-3805, 1998) synthetisiert. 4-Nitrophenylalanin wurde von Bachem (Heidelberg, (Deutschland) bezogen. D,L-3-Cyanophenylalanin und D,L-4-Cyanophenylalanin waren von Sennchemicals (Dielsdorf, Schweiz), Boc-D-3-Cyanophenylalanin und Boc-L-3-Cyanophenylalanin von Syntetech (Albany, Oregon, USA). Die N*^{a}*-Entschützung der beiden letztgenannten Verbindungen wurde in 95% TFA (Trifluoressigsäure) durchgeführt.

### Beispiel 1: Synthese von N-tert-Butyloxycarbonyl-D,L-3-cyanophenylalanin-(5)

Zu einer gerührten Lösung von D,L-(3-Cyano)phenylalanin (5 g; 26,29 mmol) in Dioxan (25 ml) und 1 M NaOH (26,3 ml) wurde (BOC)₂O (5,74 g; 26,29 mmol) in Dioxan (5 ml) zugegeben. Nach einer Stunde wurde die Lösung verdampft und der Rest zwischen AcOEt und 5%iger wäßriger KHSO₄ Lösung aufgeteilt. Die wäßrige Phase wurde dreimal mit AcOEt extrahiert, und die kombinierten organischen Phasen wurden getrocknet (über Na₂SO₄ und verdampft, wodurch ein blasses gelbes Öl erhalten wurde, das bei 4 °C kristallisierte.
Ausbeute: 6,9 g (91 %); TLC (Lösungsmittelsystem B): R_{f} 0,77; HPLC: t_{R} 8,2 min; MS *m*/*z* 291,0 (M+H)⁺, berechnetes Mᵣ = 290,1.

### Beispiel 2: Synthese von N-tert-Butyloxycarbonyl-D,L-3-hydroxyamidinophenylalanin (6)

Eine Lösung von Verbindung **5** (1 g; 3,44 mmol), Hydroxylaminhydrochlrid (359 mg ; 5,17 mmol) und KOH (483 mg; 8,6 mmol) in EtOH (50 ml) wurde unter Rückfluß über Nacht gekocht. Unlösliches KCI wurde abgefiltert, die Lösung verdampft und der Rest in Wasser (30 ml) gelöst und mit 1 M HCl auf pH 2,5 angesäuert. Die Lösung wurde zweimal mit AcOEt (20 ml) gewaschen. und anschließend wurde das Produkt fünfmal mit wassergesättigtem n-BuOH extrahiert. Die vereinigten n-BuOH-Schichten wurden verdampft.
Ausbeute: 870 mg (78 %) weißer Schaum; TLC (Lösungsmittelsystem C): R_{f} 0,62; HPLC: t_{R} 5,3 min; MS *m*/*z* = 324.0 (M+H)⁺, berechnetes Mᵣ = 323,2.

### Beispiel 3: Synthese von N-tert-Butyloxycarbonyl-D,L-3-amidinophenyl-alaninhydrochlorid (7)

Die Verbindung **6** (870 mg; 2,69 mmol) wurde in Wasser (50 ml) über 10 % Pd/C für eine Dauer von 5 h bei 50°C hydriert. Der Katalysator wurde abgefiltert und die Lösung wurde unter Zugabe von 1 M HCl (2,7 ml) bis zur Trockenheit verdampft.
Ausbeute: 710 mg (77 %); TLC (Lösungsmittelsystem C):R_{f} 0,18; HPLC: t_{R} 5,4 min; MS: *m*/*z*= 308,4 (M+H)⁺, berechnetes Mᵣ = 307,2.

### Beispiel 4: Synthese von N-tert-Butyloxycarbonyl-D,L-3-amidinophenylalanin-methylesterhydrochlorid (8)

Eine Lösung von 7 in MeOH (5 ml) wurde mit 6 M HCl auf pH 2 angesäuert und bei Raumtemperatur für 24 h gerührt. Die Lösung wurde bis zur Trockenheit verdampft.
Ausbeute: quantitativ; TLC (Lösungsmittelsystem C): R_{f} 0,41; HPLC: t, 5,9 min; MS *m*/*z* = 322,4 (M+H)⁺, berechnetes Mᵣ = 321,2.

### Beispiel 5: Synthese von D,L-3-Amidinophenylalaninmethylester-dihydrochlorid (9)

Eine Lösung von **8** (230 mg; 0,64 mmol) in 6 M HCl in Dioxan (5 ml) wurde bei Raumtemperatur gerührt. Nach 1 h wurde die Lösung bis zur Trockenheit verdampft.
Ausbeute: quantitativ; TLC (Lösungsmittelsystem B): R_{f} 0,10; MS: *m*/*z* = 222,2 (M+H)⁺, berechnetes Mᵣ = 221.1

### Beispiel 6: Synthese von N-9-Fluorenylmethyloxycarbonyl-D,L-3-amidino-phenylalaninmethylesterhydrochlorid 10)

Zu einer Lösung von Verbindung **9** (50 mg; 0,17 mmol) in DMF (500 *µ*l) wurden Fmoc-Cl (9-Fluorenylmethoxycarbonylchlorid, 44 mg; 0,17 mmol) und TEA (Triethylamin, 24 µl; 0,17 mmol) zugegeben. Nach 30 min bei Raumtemperatur wurde TEA (12 µl) zugegeben, um die Reaktion zu vervollständigen. Nach 3 h wurde das Lösungsmittel verdampft und der Rest in Wasser gelöst. Nach der Ansäuerung mit 1 M HCl auf pH 3 wurde das Produkt durch Zentrifugation gesammelt und aus AcOEt/Diisopropylether wieder ausgefällt.
Ausbeute: 60 mg (73 %); TLC (Lösungsmittelsystem A): R_{f} 0.58 ; HPLC: t_{R} 8,0 min; MS: *mlz* = 444,0 (M+H)⁺, berechnetes Mᵣ = 443,2.

### Beispiel 7: Synthese von N-1-Adamantyloxycarbonyl-D,L-3-amidinophenyl-alaninmethylesterhydrochlorid (11)

Die Verbindung **11** wurde im wesentlichen wie für Verbindung **10** beschrieben mit Adoc-F (1-Adamantyloxycarbonylfluorid) hergestellt und wurde aus AcOEt/Diisopropylether wieder ausgefällt.
Ausbeute: 86 %; TLC (Lösungsmittelsystem A): R_{f} 0,55; HPLC: t_{R} 7,6 min; MS: *m*/*z=* 400,4 (M+H)⁺, berechnetes Mᵣ = 399,2.

### Beispiel 8: Synthese von N-1-Adamantylaminocarbonyl-D,L-3-amidinophenyl-alaninmethylesterhydrochlorid (12)

Verbindung **9** (46 mg 0,156 mmol) wurde in DMF (500 *µ*l) mit 1-Adamantylisocyanat (27,7 mg; 0,156 mmol) und TEA (22 *µ*l, 0,156 mmol) 3 h lang umgesetzt. Nach Verdampfung des Lösungsmittel wurde der Rest aus Isopropanol/Diisopropylether kristallisiert.
Ausbeute: 55 mg (81 %); HPLC: t_{R} 8,7 min; MS: *m*/*z* = 399,4 (M+H)⁺ berechnetes Mᵣ = 398,2.

### Beispiel 9: Synthese von N-1-Adamantyloxycarbonyl-D,L-3-cyanophenylalanin (13)

Eine Lösung von D,L-(3-Cyano)phenylalanin (2 g; 10,5 mmol), Adoc-F (2,08 g; 10,5 mmol) und 2 M NaOH (7,8ml; 15,6 mmol) in Dioxan (50 ml) wurde 3 h lang bei Raumtemperatur gerührt. Der Rest wurde zwischen AcOEt und 5 % wäßriger KHSO₄-Lösung aufgeteilt. Die wäßrige Phase wurde dreimal mit AcOEt extrahiert, die vereinigten organischen Phasen wurden mit Salzlösung gewaschen, getrocknet (über Na₂SO₄) und verdampft. Das resultierende gelbliche Öl wurde mit Diethylether behandelt und bis zu einem weißen Schaum verdampft.
Ausbeute: 3,7 g (96 %); HPLC: t_{R} 8,7 min; TLC (Lösungsmittelsystem B): R_{f} 0,72; MS *m*/*z* = 369,5 (M+H)⁺ berechnetes Mᵣ = 368,2.

### Beispiel 10: Synthese von N-1-Adamantyloxycarbonyl-D,L-3-hydroxyamidino-phenylalaninhydrochlorid (14)

Verbindung **13** (3,7 g; 10 mmol) wurde mit Hydroxylaminhydrochlorid umgesetzt und wie für Verbindung **6** beschrieben aufgearbeitet.
Ausbeute: 3,9 g (97 %); HPLC: t_{R} 9,1 min; TLC (Lösungsmittelsystem B): R_{f} 0,66; MS m/z = 402,4 (M+H)⁺, berechnetes Mᵣ = 401,2.

### Beispiel 11: Synthese von N-1-Adamantyloxycarbonyl-D,L-3-amidinophenylalaninhydrochlorid (15)

Die katalytische Reduktion der Verbindung **13** (3,9 g; 9,7 mmol) wurde wie für Verbindung 7 beschrieben durchgeführt.
Ausbeute: 3,5 g (86 %); HPLC: t_{R} 9,4 min; MS: m/z = 386,4 (M+H)⁺, berechnetes Mᵣ = 385,2.

### Beispiel 12: Synthese von N-1-Adamantyloycarbonyl D,L-3-cyanophenylalaninpiperidid (16)

Zu einer Lösung von Verbindung **13** (300 mg; 0,814 mmol) und Piperidin (480 µl; 4,88 mmol) in Methylenchlorid (5 ml) wurde SOCl₂ (120 µl; 1,63 mmol) tropfenweise bei 0 °C unter heftigem Rühren zugegeben. Nach dem Erwärmenlassen auf Raumtemperatur und nach 2 h wurde die Reaktionsmischung mit Methylenchlorid verdünnt und mit 5 % wäßriger NaHCO₃, 5 % wäßriger KHSO₄-Lösung, Wasser und Salzlösung gewaschen und getrocknet (über Na₂SO₄). Die Lösung wurde zur Trockenheit gebracht.
Ausbeute: 240 mg (68 %); TLC (Lösungsmittelsystem D): R_{f} 0,76; MS: m/z = 436,2 (M+H)⁺ berechnetes Mᵣ = 435,3.

### Beispiel 13: Synthese von N-1-Adamantyloxylcarbonyl-D,L-3-amidinophenyl-alaninpiperididhydrochlorid (17)

Die Umsetzung von Verbindung **16** (240 mg; 0,55 mmol) mit Hydroxylaminhydrochlorid und die folgende katalytische Reduktion zur Verbindung **17** wurde wie für die Verbindungen 6 und 7 beschrieben durchgeführt.
Ausbeute: 84 mg (31 % über beide Schritte); HPLC: t_{R} 10,0 min; MS: m/z = 453,4 (M+H)⁺, berechnetes Mᵣ = 452,3.

### Beispiel 14: Synthese von N-tert-Butyloxycarbonyl-D,L-(4-amidino)phenyl-alaninhydrochlorid (18) (Vergleichsbeispiel)

Die Verbindung **18** wurde ausgehend von D,L-(4-Cyano)phenylalanin wie für das 3-substituierte Phenylalanin **7** beschrieben synthetisiert.
Ausbeute: 57 % (über 3 Schritte); HPLC: t_{R} 5,2 min; MS m/z = 308,4 (M+H)⁺, berechnetes Mᵣ = 307,2.

### Beispiel 15: Synthese von D,L-4-Amidinophenylalanindihydrochlorid (19) (Vergleichsbeispiel)

Die Entschützung der Verbindung **18** (625 mg; 2,037 mmol) wurde in 6 M HCL in Dioxan wie für Verbindung **9** beschrieben durchgeführt.
Ausbeute: 540 mg (95 %); TLC (Lösungsmittelsystem E): R_{f} 0,23; MS: m/z = 208,3 (M+H)⁺, berechnetes Mᵣ = 207,2.

### Beispiel 16: Synthese von D,L-4-Amidinophenylalaninmethylesterdihydrochlorid (20) (Vergleichsbeispiel)

Zur einer Lösung von Verbindung **17** (230 mg; 0,824 mmol) in MeOH (2 ml) wurde SOCl₂ (180 µl; 2,47 mmol) tropfenweise unter heftigem Rühren bei -70 °C zugegeben. Die Reaktionsmischung wurde auf Raumtemperatur erwärmen gelassen und 18 h lang gerührt. Das Lösungsmittel wurde verdampft, und das Produkt wurde aus EtOH/Diethylether kristallisiert.
Ausbeute: 182 mg (75 %); TLC (Lösungsmittelsystem E): R_{f} 0,54; MS: m/z = 222.4 (M+H)⁺, berechnetes Mᵣ = 221,1.

### Beispiel 17: Synthese von N-1-Adamantyloxycarbonyl-D,L-4-amidinophenylalaninmethylesterhydrochlorid (21) (Vergleichsbeispiel)

Die Verbindung **21** wurde aus Verbindung **20** mit Adoc-F wie für Verbindung **10** beschrieben hergestellt.
Ausbeute: 32 mg (43 %); HPLC: t_{R} 9,4 min; MS: m/z = 400,4 (M+H)⁺, berechnetes Mᵣ = 399,2.

### Beispiel 18: Synthese von D,L-4-Nitrophenylalaninmethylesterhydrochlorid (22) (Vergleichsbeispiel)

Zu einer eisgekühlten Lösung von (4-Nitro)phenylalanin (1 g; 4,72 mmol) in MeOH (5 ml) wurde SOCl₂ (1,27 µl; 18,88 mmol) tropfenweise zugegeben. Nach 20 h wurde das Lösungsmittel verdampft und der schwachgelbe Feststoff mit Ether gewaschen und getrocknet.
Ausbeute: 1,19 g (97 %); TLC (Lösungsmittelsystem F): R_{f} 0,70; MS: m/z = 225,2 (M+H)⁺, berechnetes Mᵣ = 224.1

### Beispiel 19: Synthese von N-1-Adamantyloxycarbonyl-D,L-4-nitrophenyl-alaninmethylester (23) (Vergleichsbeispiel)

Die Verbindung **23** wurde aus Verbindung **22** mit Adoc-F wie für Verbindung **10** beschrieben hergestellt.
Ausbeute: 725 mg (94 %); HPLC t_{R} 12,7 min; MS: m/z = 403,4 (M+H)⁺, berechnetes Mᵣ = 402,2.

### Beispiel 20: Synthese von N-1-Adamantyloxylcarbonyl-D,L-4-aminophenyl-alaninmethylester (24) (Vergleichsbeispiel)

Die Verbindung **23** (725 mg; 1,8 mmol) wurde über Pd/C in MeOH (20 ml) 2 h lang hydriert; anschließend wurde der Katalysator abgefiltert und das Lösungsmittel verdampft. Das Rohprodukt wurde über Silikagel chromatographiert (Eluent: n-Hexan/AcOEt/AcOH, 49:49:2)
Ausbeute: 556 mg(83 %) HPLC: t_{R} 14,2 min; TLC (Lösungsmittelsystem G): R_{f} 0,54; MS:m/z = 373,4 (M+H)⁺, berechnetes Mᵣ = 372,4.

### Beispiel 21: Synthese von N-1-Adamantyloxycarbonyl-D,L-4-(N^{w},N^{w'}-dibenzyloxycarbonyl)guanidinophenylalaninmethylester (25) (Vergleichsbeispiel)

Eine Lösung von Verbindung **24** (57 mg; 0,153 mmol), N,N'-Dibenzyloycarbonyl-N"-triflylguanidin (70 mg; 0,153 mmol) und TEA (21 µl; 0,153 mmol) in Methylenchlorid (500 µl) wurde in einem abgeschlossenen Reaktionsgefäß mit Schraubverschluß für drei Tage bei 50 °C gerührt. Das Lösungsmittel wurde verdampft und das Rohprodukt in AcOEt (10 ml) zweimal mit 5 % wäßriger KHSO₄, Wasser und Salzlösung gewaschen. Die organische Phase wurde getrocknet (über Na₂SO₄) und verdampft.
Ausbeute: 96 mg (92 %) eines farblosen Öles; HPLC: t_{R} 14,5 min; MS: m/z = 683,4 (M+H)⁺, berechnetes Mᵣ = 682,3.

### Beispiel 22: Synthese von N-1-Adamantyloycarbonyl-D,L-4-guandidinophenylalaninmethylesterhydrochlorid (26) (Vergleichsbeispiel)

Die Verbindung **26** wurde durch katalytische Hydrierung von Verbindung **25** (96 mg; 0,141 mmol) über Pd/C in MeOH (5 ml), daß 1 M HCl (140 µl; 0,141 mmol) enthält, erhalten. Der Katalysator wurde abgefiltert, das Lösungsmittel verdampft und der Rest aus AcOEt/Diisopropylether rekristallisiert.
Ausbeute: 53 mg (83 %); HPLC: t_{R} 9,5 min; MS m/z = 415.4 (M+H)⁺, berechnetes Mᵣ = 414,2.

### Beispiel 23: Synthese von N-1-Adamantyloxylcarbonyl-L-3-amidinophenyl-alaninmethylesterhydrochlorid (27)

Die Verbindung **27** wurde wie für Verbindung **11** beschrieben ausgehend von L-(3-Cyano)phenylalanin synthesisiert.
HPL: t_{R} 9,6 min; MS: m/z = 400,2 (M+H)⁺, berechnetes Mᵣ = 399,2.

### Beispiel 24: Synthese von N-1-Adamantyloxycarbonyl-D-3-amindinophenyl-alaninmethylesterhydrochlorid (28).

Die Verbindung **28** wurde wie für Verbindung **11** beschrieben ausgehend von D-(3-Cyano)phenylalanin synthetisiert,.
HPLC: t_{R} 8,2 min, MS: m/z = 400,4 (M+H)⁺, berechnetes Mᵣ = 399,2.

### Beispiel 25: Synthese von N-1-Adamantyloxycarbonyl-D-3-amidinophenylalaninpropylamidhydrochlorid (29)

N^{a}-Adoc-D-3-amidinophenylalaninhydrochlorid (50 mg; 0,118 mmol), n-Propylamin (29 *µ*l; 0,354 mmol) und HOBT (19 mg; 0,142 mmol) wurde in 2 ml DMF gelöst. TBTU (46 mg; 0,142 mmol) wurden zugefügt und die Reaktionsmischung wurde 3 h lang bei Raumtempertur gerührt. Nach der Lösungsmittelverdampfung im Vakuum wurde das erhaltene Öl in 20 ml Ethylacetat gelöst. Das Produkt begann sofort auszufallen und wurde durch Zentrifugation abgetrennt. Der farblose Feststoff wurde mit Ethylacetat gewaschen und im Vakuum getrocknet.
Ausbeute: 26 mg (47 %); HPLC: t_{R} 7,8 min; MS; m/z = 427,4 (M+H)⁺, berechnetes Mᵣ= 426,3.

### Beispiel 26: Synthese von N-1-Adamantyloxycarbonyl-D=3-amidinophenyl-alaninbenzylamidhydrochlorid (30).

N^{a}-Adoc-D-3-amidinophenylalaninhydrochlorid (30 mg; 0,071 mmol), Benzylamin (23 µl; 0,213 mmol) und HOBT (11 mg; 0,085 mmol) wurden in 2 ml DMF gelöst. TBTU (27 mg; 0,085 mmol) wurde zugegeben, und die Reaktionsmischung wurde bei Raumtemperatur gerührt. Nach 3 h wurde das ausgefallene Salz abgefiltert und das Lösungsmittel im Vakuum verdampft. Der Rest wurde in 10 ml Ethylacetat gelöst, mit 5 %iger wäßriger NaHCO₃-Lösung und Salzlösung gewaschen und über wasserfreiem Na₂SO₄ getrocknet. Nach der Lösungsmittelverdampfung im Vakuum wurde das Rohrprodukt in 1 ml Ethylacetat gelöst. 10 µl 6N HCl in Dioxan wurden zugegeben und das Produkt mit tert-Butylmethylether ausgefällt. Der flockige Niederschlag wurde mit Diethylether gewaschen und im Vakuum getrocknet.
Ausbeute: 16 mg (43 %); HPLC: t_{R} 10,3 min; MS: m/z = 475,2 (M+H)⁺, berechnetes Mᵣ = 474,3.

### Beispiel 27: Synthese von N-1-Adamantyloxycarbonyl-D-3-amidinophenylalanin-(2-phenyl)-1-ethylamin-hydrochlorid (31)

N^{a}-Adoc-D-3-amidinophenylalaninhydrochlorid (30 mg ; 0,071 mmol), Phenetylamin (27 *µ*l; 0,213 mmol) und HOBT (11 mg; 0,085 mmol) wurden in 2 ml DMF gelöst. TBTU (27 mg; 0.085 mmol) wurden zugegeben, und die Reaktionsmischung wurde bei Raumtemperatur gerührt. Nach 3 h war die Reaktion nicht abgeschlossen und 15 mg TBTU (0,047 mmol) wurden zugegeben und die Mischung wurde für weitere 3 h gerührt. Das Lösungsmittel wurde im Vakuum verdampft. Der Rest wurde in 10 ml Ethylacetat gelöst, dreimal mit 5 % wäßriger NaHCO₃-Lösung, 1 x mit 2 ml 0,5 N HCl gewaschen und über wasserfreiem Na₂SO₄ getrocknet. Nach der Lösungsmittelverdampfung im Vakuum wurde das Produkt aus iPrOH/DIPE ausgefüllt. Der flockige Niederschlag wurde mit Diethylether gewaschen und im Vakuum getrocknet.
Ausbeute: 10 ml (27 %); HPLC: t_{R} 6,8 min; MS. m/z = 489,4 (M+H)⁺, berechnetes Mᵣ = 488,3.

### Beispiel 27a:

Die Synthese der Verbindungen 32 bis 36 erfolgte in analoger Weise zu den oben beschrieben Herstellungsverfahren.

Die Verbindung 32 mit dem Linker -NH-CO-NH- hemmt FXa um den Faktor 3 besser als die Verbindung 31 mit dem Linker -O-CO-NH-.

### Beispiel 28: Bestimmung der Inhibierungskonstanten

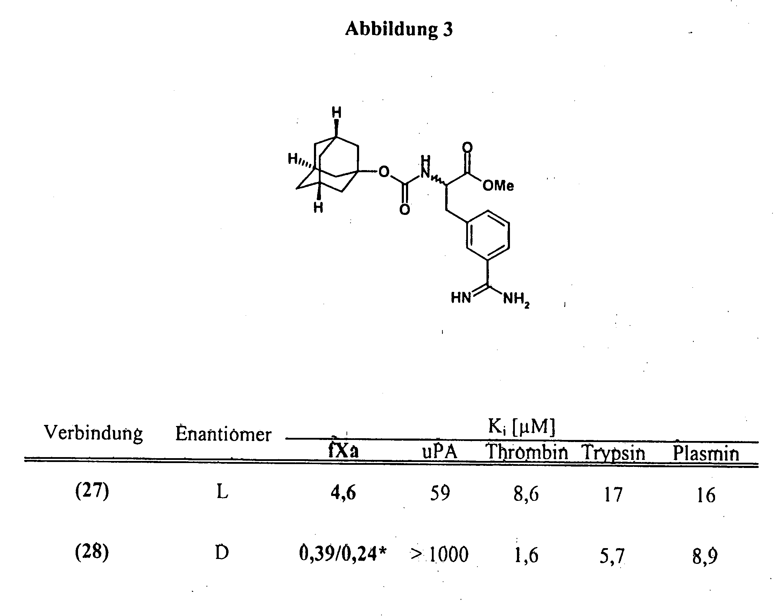

Die Messungen wurden auf einem Mikroplatten-Reader (MR 5000, Dynatech, Denkendorf, Deutschland) bei 25 °C durchgeführt. Das Testmedium bestand aus 200 *µ*l Tris-Buffer (0,05 M; 0,154 M NaCl, 5 % Ethanol, ph 8,0), 25 *µ*l wäßriger Substratlösung und 50 *µ*l Enzymlösung. Zwei Konzentrationen des Substrats sowie fünf Konzentrationen des Inhibitors wurden verwendet. Drei Minuten nach der Zugabe des Enzyms wurden 25 *µ*l Essigsäure (50 %) zugegeben, um die Reaktion zu quenchen, und die optische Dichte wurde bei 405 nm gemessen. Die Kᵢ-Werte wurden nach Dixon (M. Dixon (1953), Biochem. J. 55, 170-171)) unter Verwendung einer linearen Regression berechnet. Die in den Abbildungen 2 und 3 angegebenen Kᵢ-Werte sind Mittelwerte von mindestens drei Bestimmungen.

### Beispiel 29: Enyme und Substrate für die Kᵢ-Bestimmung

Die folgenden Enzyme und die entsprechenden Substrate wurden bei den angegebenen Endkonzentrationen verwendet:
Rinderthrombin, hergestellt nach Walsmann (P. Walsmann (1968), Pharmazie 23, 401-402) (2262 U/mg, Endkonzentration 0,45 U/mL), Substrat MeSO₂-D-Hexahydrotyrosyl-Gly-Arg-pNA (Endkonzentrationen 0,18 und 0,09 mM);
Rinder-Faktor Xₐ (5U/vial, 0,11 U/ml; Diagnostic Reagents Ltd., Thame, UK), Substrat MeSO₂-D-Nle-Gly-Arg-pNA (0.36 und 0,18 mM);
Humaner Faktor Xₐ (0,18 *µ*g/ml; Kordia Lab. Supplies, Leiden, Niederlande), Substrat wie bei Rinder-Faktor Xₐ
Human-Plasmin (0,67 CTA-U/mg, 0,06 CTA-U/mL; Behringwerke AG, Marburg, Deutschland), Substrat Tos-Gly-Pro-Lys-pNA (0,18 und 0,09 mM);
Humanes uPA (500.000 U/vial, Endkonzentration 150 U/mL; Ribosepharm GmbH Haan, Deutschland), Substrat Bz-βAla-Gly-Arg-pNA (0,18 und 0,09 mM);
Rinder-Pankreastrypsin (42 U/mg, 0,0038 U/ML; Serva, Heidelberg, Deutschland,), Substrat MeSO₂-D-Hexahydrotyrosyl-Gly-Arg-pNA (0,18 und 0,06 mM).

Die Substrate wurden von Pentapharm Ltd., Basel, Schweiz geliefert.

### Abbildungen

Abbildung 1: Strukturen und Inhibitorstärken der Faktor Xₐ-Inhibitoren 1, 2 und 3 aus dem Stand der Technik.
Abbildung 2: Derivate von 3-/4-Amidino- bzw. 4-Guanidinophanylalanin mit N "-substituiertem Carbamat oder Harnstoff.
Abbildung 3: Enantiomerenreine Derivate von 1-Adamantyloxycarbonyl-3-amidino-phenylalaninmethylester. Mit * gekennzeichnete Kᵢ-Werte entsprechen Kᵢ-Werten für humanen Faktor Xₐ.
Abbildung 4: DerivatevonN-1-Adamanthyloxycarbonyl-3-amidinophenylalanin mit C^{a}-substituiertem Amid. Mit * gekennzeichnete Kᵢ-Werte entsprechen Kᵢ-Werten für humanen Faktor Xₐ.
Abbildung 4a: Weitere Derivate von N-1-Adamanthyloxycarbonylamidinophenylalanin mit C*^{a}*-substituiertem Amid, wobei Verbindungen mit verschiedenen Resten R² sowie deren Kᵢ-Werte dargestellt sind.

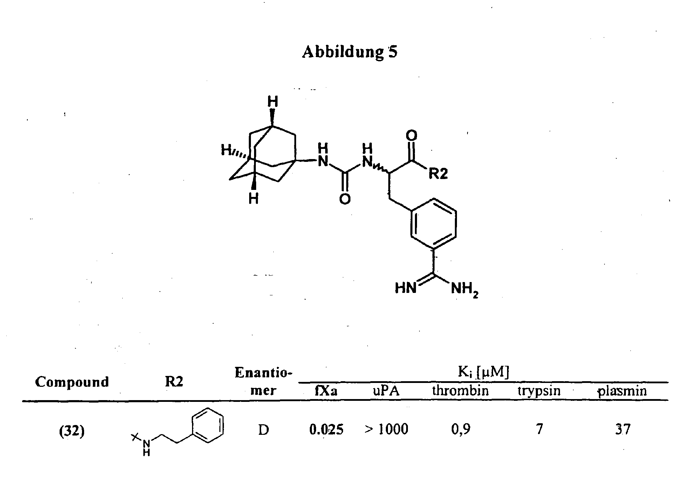

Abbildung 5: N-(1-Adamanthylaminocarbonyl)-D-3-amidinophenylalanin-(2-phenyl)-1-ethylamid und dessen Kᵢ-Werte.

## Patentansprüche

1. Verbindung mit der folgenden Strukturformel I 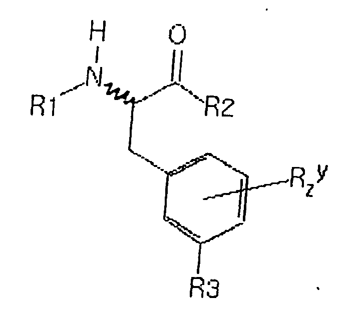 in der:
- R¹ einen direkt mit dem Phenylalaninanalogon verbundenen Linker L¹ ausgewählt aus einer Bindung, einer Gruppierung R^{x}, -CO-, -CO-NH-, -COO-, -CS-, -CS-NH-, -COS- oder -CO-CH₂-NH und eine Gruppe R⁴ umfasst, wobei R⁴ ein ein- oder mehrfach mit R^{y} substituierter oder nicht substituierter, gesättigter oder ungesättigter carbocyclischer Rest, heterocyclischer Rest oder Alkylrest ist; R^{x} C₁-C₁₀-Alkylen, C₁-C₁₀-Alkenylen oder C₁-C₁₀-Alkinylen bedeutet, welches gegebenenfalls Heteroatome in der Kette aufweist;
- R² einen direkt mit dem Phenylalaninanalogon verbundenen Linker L² ausgewählt aus -OR⁵-, -NH-R⁵-, -NH-NH-R⁵- oder -CH₂-R⁵-, wobei R⁵ ein substituierter oder nicht substituierter, gesättigter oder ungesättigter carbocyclischer, heterocyclischer oder nicht cyclischer Alkylrest oder eine Gruppe R^{X} sein kann und eine Gruppe R⁷ umfasst, wobei R⁷ ein ein- oder mehrfach mit R⁸ substituierter oder nicht substituierter C₁₋₃₀-Alkyl-, C₂₋₃₀-Alkenyl-, C₂-₃₀-Alkinyl-, C₃₋₃₀-Cycloalkyl-, C₅₋₃₀-Aryl-, C₃₋₃₀-Heteroaryl-, C₆₋₃₀-Alkaryl- oder C₄₋₃₀-Alkheteroaryl-Rest ist und R⁸ ein Amidino-, Guanidino-, Amino-, Ester-, Alkylamino-, Aminoalkyl, Cyano-, Amid- oder Hydroxylrest oder/und Halogen, -OH, -NH₂, -Formyl, -Acetyl, -OMe, -OEt, -NHMe, -NHEt, -SH, SEt, -SMe, -NMe₂, -CH₃, -CH₂OH, -CH₂CH₃, -NHOH, -COOH, -COOMe, CN, NO₂ oder -CH₂CH₃ ist,
- R³ ein Amidino- oder ein Guanidinorest an der 3-Position des aromatischen Rings des Phenylalaninrests ist und der aromatische Ring gegebenenfalls mit zusätzlichen Substituenten R^{Y}, ausgewählt aus Halogen, -OH, -NH₂, -Formyl, -Acetyl, -OMe, -OEt, -NHMe, -NHEt, -SH, SEt, SMe, -NMe₂, -CH₃, -CH₂OH -CH₂CH₃, - NHOH, -COOH, -COOMe, CN, NO₂, -CH₂CH₃, substituiert ist,
wobei z = 0 bis 4.

2. Verbindung nach Anspruch 1, wobei der Linker L¹ = -CO-, -CO-NH- oder -COO- ist.

3. Verbindung nach Anspruch 1 oder 2, wobei R⁴ Phenyl, Benzyl, Fluorenyl oder Adamantyl ist.

4. Verbindung nach einem der Ansprüche 1 bis 3, wobei R⁵ die Formel -(CH₂)ₘ- mit m = 1 bis 3 aufweist.

5. Verbindung nach einem der Ansprüche 1 bis 4, wobei R⁷ ein nicht substituierter oder mit einem oder mehreren Resten R⁸ substituierter Phenyl-, Piperidin-, Pyrrol-, Furan-, Thiophen-, Pyridin-, Naphthalin-, Anthracen- oder Indolrest ist.

6. Verbindung nach einem der Ansprüche 1 bis 5, wobei der Phenylalaninrest (R)-chiral ist.

7. Verbindung nach einem der Ansprüche 1 bis 6, ausgewählt aus N-(1-Adamantylaminocarbonyl)-D-3-amidinophenylalanin-(2-phenyl)-1-ethylamid, N-1-Adamantyloxycarbonyl-D-3-amidinophenylalanin-(2-phenyl)-1-ethylamid, N-1-Adamantyloxycarbonyl-D3-amidinophenylalaninpropylamid, oder N-1-Adamanatyloxycarbonyl-D-3-amidinophenylalaninbenzylamid.

8. Verbindung nach einem der Ansprüche 1 bis 7 in Form eines pharmazeutisch akzeptablen Salzes, insbesondere Hydrochlorid.

9. Verfahren zur Herstellung einer Verbindung nach einem der Ansprüche 1 bis 8, umfassend die folgenden Schritte:
a) Zugabe von R⁴-NCO, R⁴-NCS, X-CO-R⁴, X-SO₂-R⁴, X-CO-NH-R⁴ oder X-COOR⁴ zu D- oder L-Phenylalanin, das an der 3-Position den Substituenten R³ oder eine Vorstufe von R³ aufweist;
b) gegebenenfalls Umwandlung der Vorstufe von R³ in den Substituenten R³;
c) gegebenenfalls Zugabe von YR⁵ zu dem Reaktionsprodukt des Schritts b).

10. Pharmazeutische Zusammensetzung, umfassend eine Verbindung nach einem der Ansprüche 1 bis 8 und gegebenenfalls einen pharmazeutisch akzeptablen Träger.

11. Verwendung einer Verbindung nach einem der Ansprüche 1 bis 8 zur Herstellung eines Mittels zur antikoagulatorischen Therapie, zur Herstellung eines Antitumormittels oder als diagnostisches Mittel.

## Claims

1. Compound having the following structural formula I 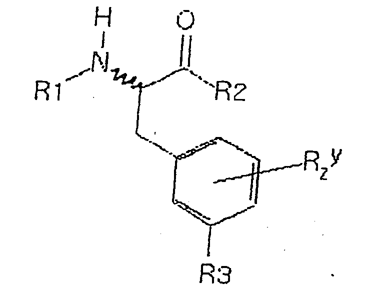 in which
- R¹ comprises a linker L¹ directly bound to the phenylalanine analogue selected from a bond, a group R^{x}, -CO-, -CO-NH-, -COO-, -CS-, -CS-NH-, -COS- or -CO-CH₂-NH and a group R⁴, R⁴ being a saturated or unsaturated carbocyclic residue, heterocyclic residue or alkyl residue which is monosubstituted or polysubstituted with R^{Y} or is unsubstituted; R^{x} denotes C₁-C₁₀ alkylene, C₁-C₁₀ alkenylene or C₁-C₁₀ alkinylene which optionally has heteroatoms in the chain;
- R² comprises a linker L² bound directly to the phenylalanine analogue selected from -OR⁵, -NH-R⁵, -NH-NH-R⁵- or -CH₂-R⁵- in which R⁵ can be a substituted or unsubstituted, saturated or unsaturated carbocyclic, heterocyclic or non-cyclic alkyl residue or a group R^{x} and a group R⁷ in which R⁷ is a C₁₋₃₀ alkyl, C₂₋₃₀ alkenyl, C₂₋₃₀ alkinyl, C₃₋₃₀ cycloalkyl, C₅₋₃₀ aryl, C₃₋₃₀ heteroaryl, C₆₋₃₀ alkaryl or C₄₋₃₀ alkheteroaryl residue which is unsubstituted or monosubstituted or polysubstituted with R⁸ and R⁸ is an amidino, guanidine, amino, ester, alkylamino, aminoalkyl, cyano, amide or hydroxyl residue or/and halogen, -OH, -NH₂, formyl, acetyl, -OMe, -OEt, - NHMe, -NHEt, -SH, SEt, -SMe, -NMe₂, -CH₃, -CH₂OH -CH₂CH₃, -NHOH, -COOH, -COOMe, CN, NO₂ or -CH₂CH₃,
- R³ is an amidino or guanidino residue at the 3 position of the aromatic ring of the phenylalanine residue and the aromatic ring is optionally substituted with additional substituents R^{y} selected from halogen, -OH, -NH₂, formyl, acetyl, -OMe, -OEt, -NHMe, -NHEt, -SH, SEt, SMe, -NMe₂, -CH₃, -CH₂OH, -CH₂CH₃, -NHOH, -COOH, -COOMe, SN, NO₂, -CH₂-CH₃ in which z = 0 to 4.

2. Compound according to claim 1, wherein the linker L¹ = -CO-, -CO-NH- or -COO-.

3. Compound according to claim 1 or 2, wherein R⁴ is phenyl, benzyl, fluorenyl or adamantyl.

4. Compound according to one of the claims 1 to 3, wherein R⁵ has the formula -CH₂)ₘ- in which m = 1 to 3.

5. Compound according to one of the claims 1 to 4, wherein R⁷ is a phenyl, piperidine, pyrrole, furan, thiophene, pyridine, naphthalene, anthracene or indole residue which is unsubstituted or substituted with one or more residues R⁸.

6. Compound according to one of the claims 1 to 5, wherein the phenylalanine residue (R) is chiral.

7. Compound according to one of the claims 1 to 6, selected from N-(1-adamantylaminocarbonyl)-D-3-amidinophenylalanine-(2-phenyl)-1-ethylamide, N-1-adamantyloxycarbonyl-D-3-amidinophenylalanine-(2-phenyl)-1-ethylamide, N-1-adamantyloxycarbonyl-D-3-amidinophenylalanine propylamide or N-1-adamantyloxycarbonyl-D-3-amidinophenylalanine benzylamide.

8. Compound according to one of the claims 1 to 7 in the form of a pharmaceutically acceptable salt and in particular a hydrochloride.

9. Process for producing a compound according to one of the claims 1 to 8 comprising the following steps:
a) adding R⁴-NCO, R⁴-NCS, X-CO-R⁴, X-SO₂-R⁴, X-CO-NH-R⁴ or X-COOR⁴ to D- or L-phenylalanine which has the substituent R³ or a precursor of R³ at the 3 position;
b) optionally converting the precursor of R³ into the substituent R³,
c) optionally adding YR⁵ to the reaction product of step b).

10. Pharmaceutical composition comprising a compound according to one of the claims 1 to 8 and optionally a pharmaceutically acceptable vehicle.

11. Use of a compound according to one of the claims 1 to 8 to produce an agent for anticoagulant therapy, for producing an antitumour agent or a diagnostic agent.

## Revendications

1. Composé de formule générale 1 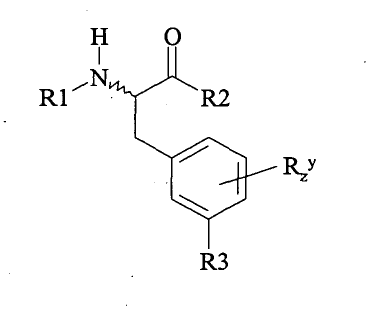 dans laquelle :
- R¹ englobe un groupe lieur L¹, directement rattaché à l'analogue de phénylalanine et choisi parmi une liaison, un groupement R^{X}, -CO-, -CO-NH-, -COO-, -CS-, -CS-NH-, -COS- ou -CO-CH₂NH, et un groupe R⁴, R⁴ étant un reste carbocyclique, un reste hétérocyclique ou un reste alkyle saturé ou insaturé mono- ou polysubstitué par R^{Y} ou non substitué ; R^{X} désigne un alkylène en C₁ à C₁₀, un alcénylène en C₁ à C₁₀ ou un alcynylène en C₁ à C₁₀ dont la chaîne présente le cas échéant des hétéroatomes ;
- R² comprend un groupe lieur L², directement rattaché à l'analogue de phénylalanine et choisi parmi -OR⁵-, -NH-R⁵-, NH-NH-R⁵- ou -CH₂-R⁵-, R⁵ pouvant être un reste alkyle carbocyclique, hétérocyclique ou non cyclique, substitué ou non substitué, saturé ou insaturé, ou un groupe R^{x}, et un groupe R⁷, R⁷ étant un reste alkyle en C₁ à C₃₀, alcényle en C₂ à C₃₀, alcynyle en C₂ à C₃₀, cycloalkyle en C₃ à C₃₀, aryle en C₅ à C₃₀, hétéroaryle en C₃ à C₃₀, alkaryle en C₆ à C₃₀ ou alkylhétéroaryle en C₄ à C₃₀ mono- ou polysubstitué par R⁸ ou non substitué, et R⁸ est un reste amidino, guanidino, amino, ester, alkylamino, aminoalkyle, cyano, amide ou hydroxyle ou/et halogène, - OH, -NH₂, -formyle, -acétyle, -OMe, -OEt, -NHMe, -NHEt, -SH, SEt, -SMe, -NMe₂, - CH₃, -CH₂OH, -CH₂CH₃, -NHOH, -COOH, -COOMe, CN, NO₂ ou -CH₂CH₃ ;
- R³ est un reste amidino ou guanidino en position 3 du noyau aromatique du reste phénylalanine et le noyau aromatique est le cas échéant substitué par d'autres substituants choisis parmi un halogène, -OH, -NH₂, -formyle, -acétyle, -OMe, -OEt, -NHMe, -NHEt, -SH, SEt, SMe, -NMe₂, -CH₃, -CH₂OH, -CH₂CH₃, - NHOH, -COOH, -COOMe, CN, NO₂, -CH₂CH₃, z valant de 0 à 4.

2. Composé selon la revendication 1, dans lequel le groupe lieur L¹ est -CO-, -CO-NH- ou -COO-.

3. Composé selon la revendication 1 ou la revendication 2, dans lequel R⁴ représente un groupe phényle, benzyle, fluorényle ou adamantyle.

4. Composé selon l'une des revendications 1 à 3, dans lequel R⁵ représente un groupe -(CH₂)ₘ-, où m vaut de 1 à 3.

5. Composé selon l'une des revendications 1 à 4, dans lequel R⁷ est un reste phényle, pipéridine, pyrrole, furane, thiophène, pyridine, naphtalène, anthracène ou indole non substitué ou substitué par un ou plusieurs restes R⁸.

6. Composé selon l'une des revendications 1 à 5, dans lequel le reste phénylalanine est l'énantiomère (R).

7. Composé selon l'une des revendications 1 à 6, choisi parmi les composés suivants :
N-(1-adamantylaminocarbonyl)-D-3-amidinophénylalanine-(2-phényl)-1-éthylamide,
N-1-adamantyloxycarbonyl-D-3-amidinophénylalanine-(2-phényl)-1-éthylamide,
N-1-adamantyloxycarbonyl-D-3-amidinophénylalaninepropylamide ou
N-1-adarnantyloxycarbonyl-D-3-amidinophénylalaninebenzylamide.

8. Composé selon l'une des revendications 1 à 7 sous forme de sel pharmaceutiquement acceptable, en particulier sous forme de chlorhydrate.

9. Procédé de préparation d'un composé selon l'une des revendications 1 à 8, comprenant les étapes consistant à :
a) ajouter du R⁴-NCO, R⁴-NCS, X-CO-R⁴, X-SO₂-R⁴, X-CO-NH-R⁴ ou X-COOR⁴ à de la D- ou L-phénylalanine présentant le substituant R³ ou un précurseur de R³ en position 3 ;
b) le cas échéant convertir le précurseur de R³ en substituant R³ ;
c) le cas échéant ajouter du YR⁵ au produit réactionnel de l'étape b).

10. Composition pharmaceutique comprenant un composé selon l'une des revendications 1 à 8 et le cas échéant un véhicule pharmaceutiquement acceptable.

11. Utilisation d'un composé selon l'une des revendications 1 à 8 pour fabriquer un agent de traitement anticoagulant, pour fabriquer un agent antitumoral, ou comme agent de diagnostic.
